(19) Europäisches Patentamt European Patent Office Office européen des brevets

(11) **EP 4 461 367 A1**

(12) **EUROPEAN PATENT APPLICATION**


(43) Date of publication: **13.11.2024 Bulletin 2024/46**

(21) Application number: **23305746.2**

(22) Date of filing: **11.05.2023**

(51) International Patent Classification (IPC): ***A61P 35/00*** *(2006.01)* ***A61K 38/00*** *(2006.01)* ***C07K 7/64*** *(2006.01)*

(52) Cooperative Patent Classification (CPC): **A61P 35/00; A61K 38/00; C07K 7/64**

(84) Designated Contracting States: **AL AT BE BG CH CY CZ DE DK EE ES FI FR GB GR HR HU IE IS IT LI LT LU LV MC ME MK MT NL NO PL PT RO RS SE SI SK SM TR**
Designated Extension States: **BA**
Designated Validation States: **KH MA MD TN**



(71) Applicants:
- **Centre National de la Recherche Scientifique 75016 Paris (FR)**
- **Institut National de la Santé et de la Recherche Médicale 75013 Paris (FR)**
- **Université de La Réunion 97744 St Denis (FR)**
- **Institut de Recherche pour le Développement 13572 Marseille Cedex 02 (FR)**

(72) Inventors:
- **APEL, Cécile 91198 GIF SUR YVETTE (FR)**
- **ROUSSI, Fanny 91198 GIF SUR YVETTE (FR)**
- **EL KALAMOUNI, Chaker 97490 SAINTE CLOTILDE (FR)**
- **LITAUDON, Marc 91198 GIF SUR YVETTE (FR)**
- **HADDAD, Juliano 97490 SAINTE CLOTILDE (FR)**
- **DESRAT, Sandy 91198 GIF SUR YVETTE (FR)**

(74) Representative: **Cabinet Nony 11 rue Saint-Georges 75009 Paris (FR)**


(54) **NEW TERNATIN-TYPE CYCLOPEPTIDES FOR USE IN THERAPY**


(57) The present invention relates to a compound of the formula (I):

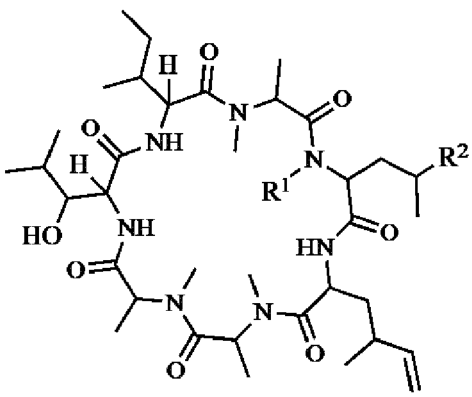

(I)

The present invention further relates to the process for extracting it, to the medicament or pharmaceutical composition containing it and to its use in therapy, in particular in the prevention and/or treatment of cancer and virus infections.


EP 4 461 367 A1

## Description

### FIELD OF THE INVENTION

Disclosed herein are ternatin-type cyclopeptides, the process for preparing them, as well as the therapeutic use thereof, in particular as antitumor and antiviral agents.

Said derivatives may in particular be useful in the treatment of virus infections, in particular RNA virus infections, even more particularly infections caused by several polarity-positive and negative single-stranded RNA viruses, such as Zika (ZIKV), Chikungunya (CHIKV), Dengue (DENV), Ross River (RRV), and *Coronaviridae,* including SARS-CoV-2.

### BACKGROUND

As used herein, ternatin is a heptacyclopeptide which was first isolated from cultured fluids of *Didymocladium ternatum sacc.* in 1974, having the CAS number 148619-41-4, also called 1,4,7,10,13,16,19-Heptaazacycloheneicosane or (-)-Ternatin. Antibiotic activities were first claimed. Ternatin and ternatin derivatives were then developed for their anti-adipogenesis effect as well as their cytotoxic activity as in document WO2010/062159. More recently, further ternatin derivatives were designed in J. D. Carelli et al., eLife 2015, 4, e10222 and their cytotoxic activity demonstrated.

There is a permanent need to find out new drugs for preventing and/or treating cancers and/or to increase the efficacy of existing anti-cancer drugs.

In addition, depending on the nature of the targeted virus, therapeutic molecules can be designed which may interfere with one or more of the mechanisms of the viruses based on their life cycle, which includes six basic steps: attachment, penetration, uncoating, replication, assembly and release.

The type of genome (single-stranded, double-stranded, RNA, DNA...) characterizes dramatically this replication step. Considering the implications of a given type of genome in the replication step, the Baltimore classification of viruses was developed. This classification clusters viruses into families (or "*groups*") depending on their type of genome. The present virus classification, as in 2018, comprises seven different groups:

- Group I: double-stranded DNA viruses (dsDNA);
- Group II: single-stranded DNA viruses (ssDNA);
- Group III: double-stranded RNA viruses (dsRNA);
- Group IV: (+)strand or sense RNA viruses ((+)ssRNA);
- Group V: (-)strand or antisense RNA viruses ((-)ssRNA);
- Group VI: single-stranded RNA viruses having DNA intermediates (ssRNA-RT);
- Group VII: double-stranded DNA viruses having RNA intermediates (dsDNA-RT).

In one embodiment, groups within the virus families particularly focused in the framework of the present invention are the ones encompassing RNA viruses, especially single-stranded RNA viruses, with positive polarity and negative polarity and more specifically RNA viruses belonging to group IV or V of the Baltimore classification.

Among the single-stranded RNA viruses with positive polarity, the following may be cited: *picornaviruses* virus family encompassing the Hepatitis A virus, the enteroviruses, rhinoviruses, the poliovirus, and foot-and-mouth virus, *flaviviruses* virus family encompassing the dengue virus, the yellow fever virus, the West Nile virus, the Japanese encephalitis virus, the Zika virus, the Usutu virus and the Tick-Borne encephalitis virus, *alphaviruses* virus family encompassing the Chikungunya virus, the Mayaro virus, the Ross River virus, and the Venezuelan Equine Encephalitis virus, *hepaciviruses* virus family encompassing the Hepatitis C virus, *hepeviruses* virus family encompassing the Hepatitis E virus and *coronaviruses* virus family encompassing HCoV, such as HCoV-229E and SARS virus such as the SARS-CoV and SARS-CoV-2.

Among the single-stranded RNA viruses with negative polarity, the following may be cited: measles virus, *Filoviridae* virus family encompassing the Ebola virus, the *Paramyxoviridae* family encompassing the Respiratory Syncytial virus (RSV), the *Rhabdoviridae* family, the *Orthomyxoviridae* family encompassing the Influenzavirus A, Influenzavirus B and Influenzavirus C.

These viruses are the cause of diseases that can lead to serious sequelae with a mortality rate ranging from 0.5 to 3%. Only symptomatic treatments exist for these diseases, no antiviral treatment is available.

There is a permanent need to find new compounds able to treat virus infections, in particular RNA virus infections, such as RNA virus infections from group IV or V. There is in particular a need to find new treatments for infections caused by Zika (ZIKV), Dengue (DENV), West Nile virus (WNV), Japanese encephalitis, Tick-borne encephalitis, Chikungunya (CHIKV), Ross River (RRV), Mayaro, measles virus or SARS-CoV-2.

## SUMMARY OF THE INVENTION

The inventors have now found that the compounds as defined in formula (I) hereinafter has such a cytotoxic and antiviral activity.

Said compound of formula (I) may thus be useful in the treatment and/or prevention of cancer and of virus infections, in particular RNA virus infections, such as RNA virus infections from group IV or V, and more particularly from group (IV), such as infections caused by Zika (ZIKV), Chikungunya (CHIKV), Dengue (DENV), Ross River (RRV), and *Coronaviridae,* including SARS-CoV-2.

According to a first aspect, the present invention therefore relates to a compound of formula (I)

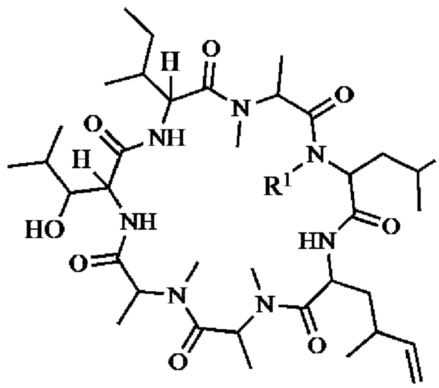
(I),

wherein it is selected from the following compounds:

- compound **C1** wherein $R^1$ is a methyl group and $R^2$ is a methyl group,
- compound **C2** wherein $R^1$ is hydrogen atom and $R^2$ is a methyl group,
- compound **C3** wherein $R^1$ is a methyl group and $R^2$ is an ethyl group,
- compound **C4** wherein $R^1$ is a methyl group and $R^2$ is a hydroxymethyl group,
- compound **C5** wherein $R^1$ is a methyl group and $R^2$ is a 1-hydroxyethyl group,
- compound **C6** wherein $R^1$ is a methyl group and $R^2$ is a hydroxyformyl methyl group, and
- compound **C7** wherein $R^1$ is a methyl group and $R^2$ is a 1-hydroxyformyl ethyl group.

Herein is further provided a compound of formula (I) as defined above, for use as a medicament.

Herein is also provided a compound of formula (I) as defined above, for use in the treatment and/or prevention of cancer and of virus infections, in particular RNA virus infections, such as RNA virus infections from group IV or V, in particular infections caused by *picornaviruses* virus family encompassing the Hepatitis A virus, the enteroviruses, rhinoviruses, the poliovirus, and foot-and-mouth virus, by *flaviviruses* virus family encompassing the dengue virus, the yellow fever virus, the West Nile virus, the Japanese encephalitis virus, the Zika virus, the Usutu virus and the Tick-Borne encephalitis virus, *alphaviruses* virus family encompassing the Chikungunya virus, the Mayaro virus, the Ross River virus, and the Venezuelan Equine Encephalitis virus, by *hepaciviruses* virus family encompassing the Hepatitis C virus, by *hepeviruses* virus family encompassing the Hepatitis E virus, by *coronaviruses* virus family encompassing HCoV, such as HCoV-229E, and SARS virus such as the SARS-CoV and SARS-CoV-2, by measles virus, by *Filoviridae* virus family encompassing the Ebola virus, by *Paramyxoviridae* virus family encompassing the Respiratory Syncytial virus (RSV), by *Rhabdoviridae* virus family or by *Orthomyxoviridae* virus family encompassing the Influenzavirus A, Influenzavirus B and Influenzavirus C, and more particularly infections caused by Zika (ZIKV), Dengue (DENV), West Nile virus (WNV), Japanese encephalitis, Tick-borne encephalitis, Chikungunya (CHIKV), Ross River (RRV), Mayaro, measles virus or SARS-CoV-2.

Herein is also provided the use of a compound of formula (I) as defined below for the manufacture of a medicament, in particular a medicament for the treatment and/or prevention of cancer and of virus infections, in particular RNA virus infections, such as RNA virus infections from group IV or V, in particular infections caused by *picornaviruses* virus family encompassing the Hepatitis A virus, the enteroviruses, rhinoviruses, the poliovirus, and foot-and-mouth virus, by *flaviviruses* virus family encompassing the dengue virus, the yellow fever virus, the West Nile virus, the Japanese encephalitis virus, the Zika virus, the Usutu virus and the Tick-Borne encephalitis virus, *alphaviruses* virus family encompassing the Chikungunya virus, the Mayaro virus, the Ross River virus, and the Venezuelan Equine Encephalitis virus, by *hepaciviruses* virus family encompassing the Hepatitis C virus, by *hepeviruses* virus family encompassing the Hepatitis E virus, by *coronaviruses* virus family encompassing HCoV, such as HCoV-229E, and SARS virus such as

the SARS-CoV and SARS-CoV-2, by measles virus, by *Filoviridae* virus family encompassing the Ebola virus, by *Paramyxoviridae* virus family encompassing the Respiratory Syncytial virus (RSV), by *Rhabdoviridae* virus family or by *Orthomyxoviridae* virus family encompassing the Influenzavirus A, Influenzavirus B and Influenzavirus C, and more particularly infections caused by Zika (ZIKV), Dengue (DENV), West Nile virus (WNV), Japanese encephalitis, Tick-borne encephalitis, Chikungunya (CHIKV), Ross River (RRV), Mayaro, measles virus or SARS-CoV-2.

Herein is finally provided a pharmaceutical composition comprising a compound of formula (I) and at least one pharmaceutically acceptable excipient, for use in the treatment and/or prevention of cancer and of virus infections, in particular RNA virus infections, such as RNA virus infections from group IV or V, in particular infections caused by *picornaviruses* virus family encompassing the Hepatitis A virus, the enteroviruses, rhinoviruses, the poliovirus, and foot-and-mouth virus, by *flaviviruses* virus family encompassing the dengue virus, the yellow fever virus, the West Nile virus, the Japanese encephalitis virus, the Zika virus, the Usutu virus and the Tick-Borne encephalitis virus, *alphaviruses* virus family encompassing the Chikungunya virus, the Mayaro virus, the Ross River virus, and the Venezuelan Equine Encephalitis virus, by *hepaciviruses* virus family encompassing the Hepatitis C virus, by *hepeviruses* virus family encompassing the Hepatitis E virus, by *coronaviruses* virus family encompassing HCoV, such as HCoV-229E and SARS virus such as the SARS-CoV and SARS-CoV-2, by measles virus, by *Filoviridae* virus family encompassing the Ebola virus, by *Paramyxoviridae* virus family encompassing the Respiratory Syncytial virus (RSV), by *Rhabdoviridae* virus family or by *Orthomyxoviridae* virus family encompassing the Influenzavirus A, Influenzavirus B and Influenzavirus C, and more particularly infections caused by Zika (ZIKV), Dengue (DENV), West Nile virus (WNV), Japanese encephalitis, Tick-borne encephalitis, Chikungunya (CHIKV), Ross River (RRV), Mayaro, measles virus or SARS-CoV-2.

In particular, the invention relates to a medicament comprising a compound of formula (I) for use in the treatment and/or prevention of cancer and of virus infections, in particular RNA virus infections, such as RNA virus infections from group IV or V in particular infections caused by *picornaviruses* virus family encompassing the Hepatitis A virus, the enteroviruses, rhinoviruses, the poliovirus, and foot-and-mouth virus, by *flaviviruses* virus family encompassing the dengue virus, the yellow fever virus, the West Nile virus, the Japanese encephalitis virus, the Zika virus, the Usutu virus and the Tick-Borne encephalitis virus, *alphaviruses* virus family encompassing the Chikungunya virus, the Mayaro virus, the Ross River virus, and the Venezuelan Equine Encephalitis virus, by *hepaciviruses* virus family encompassing the Hepatitis C virus, by *hepeviruses* virus family encompassing the Hepatitis E virus, by *coronaviruses* virus family encompassing HCoV, such as HCoV-229E, and SARS virus such as the SARS-CoV and SARS-CoV-2, by measles virus, by *Filoviridae* virus family encompassing the Ebola virus, by *Paramyxoviridae* virus family encompassing the Respiratory Syncytial virus (RSV), by *Rhabdoviridae* virus family or by *Orthomyxoviridae* virus family encompassing the Influenzavirus A, Influenzavirus B and Influenzavirus C, and more particularly infections caused by Zika (ZIKV), Dengue (DENV), West Nile virus (WNV), Japanese encephalitis, Tick-borne encephalitis, Chikungunya (CHIKV), Ross River (RRV), Mayaro, measles virus or SARS-CoV-2.

## DEFINITIONS

As used herein, the term "*patient*" refers to either an animal, such as a valuable animal for breeding, company or preservation purposes, or preferably a human or a human child, which is afflicted with, or has the potential to be afflicted with, one or more diseases described herein.

In particular, as used in the present application, the term "*patient*" refers to a mammal such as a rodent, cat, dog, primate or human, preferably said subject is a human.

The identification of those patients who are in need of treatment of herein-described diseases is well within the ability and knowledge of one skilled in the art. A veterinarian or a physician skilled in the art can readily identify, by the use of clinical tests, physical examination, medical/family history or biological and diagnostic tests, those patients who are in need of such treatment.

In the context of the invention, the term "*treating*" or "*treatment*", as used herein, means preventing, reversing, alleviating, inhibiting the progress of, or preventing the disease and its resulting cognitive, motor or metabolic changes.

Therefore, the term "*treating*" or "*treatment*" encompasses within the framework of the present invention the improvement of medical conditions of patients suffering from the diseases as described herein, in particular in the paragraph "PATHOLOGIES".

As used herein, an "*effective amount*" refers to an amount of a compound of the present invention which is effective in preventing, reducing, eliminating, treating or controlling the symptoms of the herein-described diseases.

The term "*controlling*" is intended to refer to all processes wherein there may be a slowing, interrupting, arresting, or stopping of the progression of the diseases described herein, but does not necessarily indicate a total elimination of all disease and condition symptoms, and is intended to include prophylactic treatment.

The term "*effective amount*" includes *"prophylaxis-effective amount"* as well as *"treatment-effective amount".*

The term *"preventing",* as used herein, means reducing the risk of onset or slowing the occurrence of a given phenomenon, namely in the present invention, a disease as described herein.

As used herein, *"preventing"* also encompasses *"reducing the likelihood of occurrence"* or *"reducing the likelihood of reoccurrence"*.

The term *"prophylaxis-effective amount"* refers to a concentration of compound of this invention that is effective in inhibiting, preventing, decreasing the likelihood of occurrence of anyone of the herein described diseases.

Likewise, the term *"treatment-effective amount"* refers to a concentration of compound that is effective in treating the herein described diseases.

As used herein, the term *"pharmaceutically acceptable"* refers to those compounds, materials, excipients, compositions or dosage forms which are, within the scope of sound medical judgment, suitable for contact with the tissues of human beings and animals without excessive toxicity, irritation, allergic response or other problem complications commensurate with a reasonable benefit/risk ratio.

As used herein the term "cancer", and unless stated otherwise, may relate to any disorder associated with abnormal cell growth, which thus includes malignant tumors and benign tumors, metastatic tumors and non-metastatic tumors, solid tumors and non-solid tumors, such as Blood-Related Cancers which may thus include Leukaemia, Lymphoma and Myeloma; it may also relate to Central Nervous System (CNS) cancers and non-CNS cancers. Unless stated otherwise, the term *"cancer"* also encompasses juvenile and non-juvenile cancers, Recurrent and Non-Recurrent cancers as well as cancer relapses.

As used herein, a *"viral infection or related condition"* refers to an infection of condition related to a virus, more particularly said virus having an RNA genome, and especially an RNA virus belonging to group IV or V according to the Baltimore classification. Viruses may be further classified in distinct families, orders and genus.

For reference, the content of the *"Baltimore classification"* which is reported herein further references to the virus taxonomy as set forth in the database of the 2017 International Committee of Taxonomy of Viruses (ICTV) as released online in October 2020 at https://talk.ictvonline.org/taxonomy/. This taxonomy is incorporated herein in its entirety.

The term *"pharmaceutically acceptable carrier, adjuvant, or vehicle"* may refer to any pharmaceutically acceptable excipient, such as a non- toxic carrier, adjuvant, or vehicle that does not destroy the pharmacological activity of the compound with which it is formulated.

**FIGURES LEGENDS**

**FIG 1:** COSY spectrum of **C1** as defined herein after in $CD_3CN$ at 700 MHz.
**FIG 2:** TOCSY spectrum of **C1** as defined herein after in $CD_3CN$ at 700 MHz.
**FIG 3:** HSQC-TOCSY spectrum of **C1** as defined herein after in $CD_3CN$ at 175 and 700 MHz.
**FIG 4:** HSQC spectrum of **C1** as defined herein after in $CD_3CN$ at 175 and 700 MHz.
**FIG 5:** HMBC spectrum of **C1** as defined herein after in $CD_3CN$ at 175 and 700 MHz.
**FIG 6:** ROESY spectrum of **C1** as defined herein after in $CD_3CN$ at 700 MHz.
**FIG 7:** ESI-MS/MS spectrum of **C1** as defined herein after at 30 eV.
**FIG 8:** MS/MS fragmentation patterns of **C1** as defined herein after.
**FIG 9:** Antiviral effect of compound **C1** against different viruses of medical interest (example 2.1 herein after). (A) A549 cells infected with $ZIKV^{GFP}$; (B) Vero E6 cells infected with $RRV^{GFP}$; (C) Vero E6 cells infected with DENV.
**FIG 10:** Antiviral effect of compound **C1** against coronaviruses (example 2.2 herein after). (A) Huh7 cells infected with HCoV-229E; (B) Huh7 cells infected with SARS-CoV-2.
**FIG 11:** Compound **C1** targets late stages of viral infection (example 2.3 herein after). (A) Schematic representation of synchronized $ZIKV^{GFP}$ infection and compound **C1** treatment assays in A549 cells; (B) GFP-expression in ZIKV-infected A549 cells under the conditions shown in (A); (C) GFP-expression in A549 cells incubated 1 h with $ZIKV^{GFP}$ in presence of compound **C1** added at different time points post-infection.
**FIG 12:** Evaluation of compound **C1** in a preclinical model of human epithelial cells (example 2.4 herein after).
**FIG 13:** *In vivo* evaluation of compound **C1** in a zebrafish model (example 2.5.2 herein after).

## DETAILED DESCRIPTION OF THE INVENTION

### Compounds of formula (I)

The inventors have surprisingly found that the compounds of formula (I) as disclosed herein after is useful for preventing and/or treating of cancer and of virus infections, in particular RNA virus infections, such as RNA virus infections from group IV or V, in particular infections caused by Zika (ZIKV), Dengue (DENV), West Nile virus (WNV), Japanese encephalitis, Tick-borne encephalitis, Chikungunya (CHIKV), Ross River (RRV), Mayaro, measles virus or SARS-CoV-2.

The chemical structures of the compounds of formula (I) of the invention are illustrated in the following Table I:

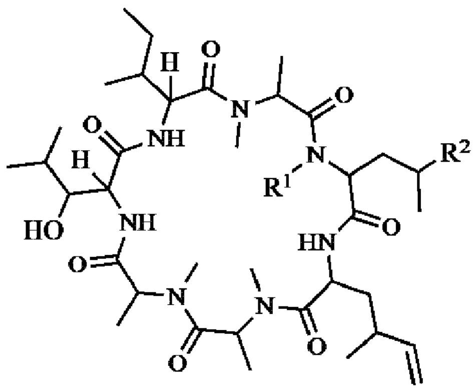

(I)

**Table I**

| No | $R^1$ | $R^2$ |
|---|---|---|
| **C1** | $CH_3$ | $CH_3$ |
| **C2** | H | $CH_3$ |
| **C3** | $CH_3$ | $CH_2CH_3$ |
| **C4** | $CH_3$ | $CH_2OH$ |
| **C5** | $CH_3$ | $CHOHCH_3$ |
| **C6** | $CH_3$ | $CH_2OCHO$ |
| **C7** | $CH_3$ | $CHCH_3OCHO$ |
| **C1** may also be called comptonellin A,<br>**C2** may also be called comptonellin B,<br>**C3** may also be called comptonellin C,<br>**C4** may also be called comptonellin D,<br>**C5** may also be called comptonellin E,<br>**C6** may also be called comptonellin F, and<br>**C7** may also be called comptonellin G. | | |

In one embodiment, herein is provided a compound selected from compounds **C1, C3, C4, C5, C6** or **C7.**

Tables 1, 2, 3 and 4 as present in Example 1 herein after illustrate the spectroscopic data of said compounds **C1** to **C7** which were thus fully characterized.

The compounds of formula (I) or compounds **C1** to **C7** can be isolated by bioassay-guided fractionation as illustrated in example 1. The fractionation and purification techniques include liquid-liquid extractions and/or chromatographic methods that separate compounds on the basis of their physicochemical properties. Liquid chromatographic methods, such as normal or reversed-phase liquid chromatography and High Performance Liquid Chromatography (HPLC), are generally employed. Each fraction resulting from the fractionation process is evaluated in a bioassay system. Active fractions undergo further cycles of separation and assaying until pure active compound is isolated. The compounds of formula (I) can also be isolated by Mass Spectrometry- (MS) or Nuclear Magnetic Resonance- (NMR) guided fractionation.

Herein is thus provided the process for isolation of the compounds of formula (I) as defined above, wherein they are extracted from species selected from the genera *Comptonella, Dutaillyea* and *Medicosma,* in particular from *Comptonella drupacea,* more particularly from their bark, leaves and/or fruits, for example bark.

In one embodiment, the compounds of formula (I) can be extracted from bark, leaves and to a lesser extent from fruits of *Comptonella drupacea* (Labill.) Guillaumin. The compounds of formula (I) can also be extracted from some other species of the genera *Comptonella, Dutaillyea* and *Medicosma.*

In another embodiment, said *Comptonella drupacea* can be collected in « Réserve spéciale de faune du col

d'Amieu et Table Unio », South Province, New Caledonia.

A corresponding voucher specimen POU-0234, collected on May 22 2008, has been deposited at the Herbarium of the IRD Center, Nouméa, New Caledonia as illustrated in example 1.

In another embodiment, bark, leaves and/or fruits, in particular bark, is dried at a temperature equal or below 40 °C, in particular ranging from 30 to 40 °C, followed by a grinding step. The grinding step may be performed for example with a mill, such as sold by the company Mill under trademarks hammer mill or Blade disc. Rotor, turbine mills can alternatively be used for such grinding step. In one embodiment, the drying step is performed using a hot air generator. Air- or heat lamp-drying can alternatively be used. The obtained dried and ground bark, leaves and/or fruits, in particular bark, may be stored at room temperature before being used for the following steps.

In one embodiment, the obtained dried and ground bark, leaves and/or fruits, in particular bark, is extracted with a solvent, such as ethyl acetate (EtOAc), dichloromethane, methanol, ethanol, acetone, diethyl ether or mixture thereof, in particular ethyl acetate.

In another embodiment, said extraction is followed by a concentration step or concentration *in vacuo.* A crude extract is thus obtained.

In another embodiment said crude extract is submitted to an isolation step, in particular performed by using a silica gel column flash chromatography. Said chromatography may be performed by using various solvent, such as *n*-hexane, cyclohexane, *n*-heptane, dichloromethane, diethyl ether, methanol (MeOH), ethanol, or acetone. For example, a gradient of *n*-heptane/EtOAc/MeOH of increasing polarities may be used. Typically, the volumetric flow may range between 10 and 250 $mL.min^{-1}$, in particular between 50 and 100 $mL.min^{-1}$.

The purification may typically be performed in 5 to 50 Column Volumes (CV), in particular between 16 and 32 CV. According to their Thin Layer Chromatography (TLC) profiles, 12 fractions of increasing polarities may then be obtained, F1-F12. The most polar fractions, F11 and F12, for example obtained by using EtOAc and MeOH, are the fractions containing the compounds of formula (I) as defined above. Said fractions may be identified by the man skilled in the art according to known techniques.

In another embodiment, said most polar, in particular fractions F11 and F12, are purified by preparative liquid chromatography, such as High Performance Liquid Chromatography (HPLC). In another embodiment, the preparative HPLC may be performed by using a mixture of aqueous buffer/organic solvent in a *ratio* varying from 90:10 to 10:90. Typically, the aqueous buffer may be water, water acidified with trifluoroacetic acid, acetic acid, or formic acid, or aqueous ammonium acetate buffer. The organic solvent may be acetonitrile ($CH_3CN$), ethanol, MeOH acidified or not. In particular a mixture of ammonium acetate buffer/$CH_3CN$ can be used. In another embodiment, the volumetric flow may range between 5 and 100 $mL.min^{-1}$, in particular between 10 and 30 $mL.min^{-1}$.

When using a preparative HPLC with Nucleodur $C_{18}$ using 10 mM ammonium acetate buffer/$CH_3CN$, 90:10 to 10:90 at 21 $mL.min^{-1}$, the retention time is for example 30.5 min for **C1** as defined herein above. With 10 mM ammonium acetate buffer/$CH_3CN$, 50:50 to 30:70 at 21 mL $min^{-1}$, the retention times are for example 15.8 min for C2, 25.3 for **C3** as defined herein above. With 10 mM ammonium acetate buffer/$CH_3CN$, 50:50 to 30:70 at 21 mL $min^{-1}$, the retention times are for example 15.4 min for **C4,** 17.5 min for **C5,** 26.5 min for **C6** or 29.7 for **C7** as defined herein above.

## PATHOLOGIES

### *Cancer*

According to one embodiment, the following cancers may be cited: head and neck cancer, stomach cancer, breast cancer, basal and squamous skin cell cancer, liver cancer, kidney cancer, brain cancer, lung cancer, pancreatic cancer, eye cancer, gastrointestinal cancer, colorectal cancer, oesophageal cancer, bladder cancer, gall bladder cancer, thyroid cancer, melanoma, myeloma, leukemia, lymphoma, uterine/cervical cancer, ovarian cancer, bone cancer and renal cancer.

According to another embodiment, the following cancers may be cited: stomach cancer, breast cancer, basal and squamous skin cell cancer, liver cancer, kidney cancer, lung cancer, pancreatic cancer, gastrointestinal cancer, colorectal cancer, bladder cancer, gall bladder cancer, melanoma, myeloma, leukemia, lymphoma, uterine/cervical cancer, ovarian cancer, bone cancer and renal cancer, and more particularly stomach cancer, liver cancer, lung cancer, pancreatic cancer, bladder cancer, melanoma, myeloma, leukemia and lymphoma.

### *Virus infections*

Among single-stranded positive RNA viruses of medical interest, one may cite the *picornaviruses* with Hepatitis A virus, enteroviruses, rhinoviruses, poliovirus, and foot-and-mouth virus, *flaviviruses* with dengue virus, yellow fever, West Nile virus, Japanese encephalitis virus, Zika virus, Usutu virus and Tick-Borne encephalitis, *alphaviruses* with Chikungunya viruse, Mayaro virus, Ross River virus, and Venezuelan Equine Encephalitis virus, *hepaciviruses* with

Hepatitis C virus, *hepeviruses* with Hepatitis E virus and *coronaviruses* with HCoV, such as HCoV-229E, and SARS virus such as SARS-CoV and SARS-CoV-2.

Alphaviruses may in particular be considered by the invention and pertain to the Group IV RNA viruses and the *Togaviridae* family, which can be defined as positive-sense single-stranded RNA viruses or (+)ssRNA viruses. Their order is "*Unassigned*" according to the Virus Taxonomy of 2017. The *Togaviridae* family includes the *Alphavirus* and *Rubivirus* genus.

Examples of Alphaviruses which are considered by the invention include: Barmah Forest virus, Chikungunya virus, Mayaro virus, O'nyong'nyong virus, Ross River virus, Semliki Forest virus, Una virus, Eastern equine encephalitis virus, Tonate virus, Venezuelan equine encephalitis virus and Wester equine encephalitis virus.

Most preferably, an alphavirus infection or alphavirus related condition, according to the invention, is a Chikungunya virus infection or Chikungunya virus-related condition.

More particularly, Chikungunya virus (CHIKV) is an RNA virus which pertains to the alphavirus genus which in turn belongs to the *Togaviridae* family, *i.e.* Group IV from the Baltimore classification. Chikungunya is a mosquito-borne viral disease first described during an outbreak in southern Tanzania in 1952. CHIKV is an enveloped, positive sense, single-stranded RNA virus with a genome of approximately 12 kb nucleotides long. The genome of CHIKV is organized as follows: 5'-cap-nsPl-nsP2-nsP3-nsP4-(junction region)-C-E3-E2-6k-El-poly(A)-3', in which the first four proteins (nsPl-4) are nonstructural proteins, and the structural proteins are the capsid (C) and the envelope proteins I. There is no distinct serotypic difference among CHIKV isolated from Africa, Asia and the islands of the Indian Ocean. Phylogenetic analyses based on El gene sequences can group CHIKV into three genotypes (lineages): Asian, east/central/south African (ECSA), and West African. The Asian genotype differed from the ECSA and West African genotypes by nucleotide levels of -5% and -15%, respectively. The African genotypes (ECSA versus West African) were -15% divergent. The amino acid identities across the three genotypes varied from 95.2 to 99.8%.

Chikungunya virus may cause outbreaks associated with severe morbidity.

Chikungunya is a viral disease transmitted to humans by infected mosquitoes. Both *Ae. aegypti* and *Ae. albopictus* have been implicated in large outbreaks of Chikungunya. Whereas *Ae. aegypti* is confined within the tropics and sub-tropics, *Ae. albopictus* also occurs in temperate and even cold temperate regions. In recent decades, *Ae. albopictus* has spread from Asia to become established in areas of Africa, Europe and the Americas.

After infection with Chikungunya virus, there is an incubation period lasting 2-4 days on average, followed by disease symptoms. Among such symptoms, fever and severe joint pain may be cited. Other symptoms include muscle pain, headache, nausea, back pain, fatigue, myalgia and rash. Severe clinical manifestations of Chikungunya infection can also occur, for example, haemorrhagic fever, conjunctivitis, photophobia, hepatitis, stomatitis. Neurologic manifestations such as encephalitis, febrile seizures, meningeal syndrome and acute encephalopathy were also reported.

Joint pain is often debilitating and can vary in duration.

The proximity of mosquito breeding sites to human habitation is a significant risk factor for Chikungunya.

The distribution of Chikungunya virus mainly occurs in Africa, India and South Eastern Asia. In recent decades, mosquito vectors of Chikungunya have spread to Europe and the Americas. In 2007, disease transmission was reported for the first time in a localized outbreak in north-eastern Italy. Outbreaks have since been recorded in France and Croatia.

Dengue viruses which present various serotypes, may also be considered by the invention and pertain to the Group IV RNA viruses and the *Flaviviridae* family, which can be defined as a positive-sense single-stranded RNA or (+)ss RNA viruses. More particularly Dengue virus, is a (+)ssRNA virus belonging to group IV of the Baltimore classification. It is part of the *Flavivirus* genus, which belongs to the *Flaviviridae* family. Other viruses pertaining to the *Flaviviridae* family are Zika virus, Japenese encephalitis virus, Usutu virus, West Nile virus, Tick-Borne virus and yellow fever virus.

An acute respiratory disease was further recently found to be caused by a novel coronavirus (SARS-CoV-2, previously known as 2019-nCoV), also referred herein as the coronavirus disease 2019 (COVID-19), which belongs to the *Coronaviridae* family and which part of the group IV of the Baltimore classification.

This coronavirus shows sustained human-to-human transmission, along with many exported cases across the globe. World Health Organization (WHO) has officially declared the COVID-19 pandemic as a public health emergency of international concern. The novel coronavirus uses the same receptor, angiotensin-converting enzyme 2 (ACE2) as that for Severe Acute Respiratory Syndrome (SARS)-CoV, and mainly spreads through the respiratory tract. The elderly and people with underlying diseases are susceptible to infection and prone to serious outcomes, which may be associated with acute respiratory distress syndrome (ARDS) and cytokine storm.

As used herein, the term *"Coronaviridae"* refers to the corresponding family of RNA viruses belonging to the group IV of the Baltimore classification, which is itself part of the *Cornidovirineae* suborder and of the *Nidovirales* Order. The *Coronaviridae* family includes both the *Letovirinae* and *Orthocoronavirinae* subfamilies.

As used herein, the term *"Letovirinae "* refers to the corresponding family of the Baltimore classification, which includes the *Alphaletovirus* genus, the *Milecovirus* subgenus, which includes (in a non-exhaustive manner) the *Microhyla letovirus 1* species.

As used herein, the term "*Orthocoronavirinae*" refers to the corresponding family of the Baltimore classification,

which includes the *Alphacoronavirus, Betacoronavirus, Deltacoronavirus*, and *Gammacoronavirus* genus.

As used herein, the term *"Alphacoronavirus"* refers to the corresponding family of the Baltimore classification, which includes the *Colacovirus, Decacovirus, Duvinacovirus, Luchacovirus, Minacovirus, Minunacovirus, Myotacovirus, Myctacovirus, Pedacovirus, Rhinacovirus, Setracovirus,* and *Tegacovirus* subgenus. In a non-exhaustive manner, this includes the following species: *Bat coronavirus CDPHE15, Bat coronavirus HKU10, Rhinolophus ferrumequinum alphacoronavirus HuB-2013, Human coronavirus 229E (HCoV-229E), Lucheng Rn rat coronavirus, Ferret coronavirus, Mink coronavirus 1, Miniopterus bat coronavirus 1, Miniopterus bat coronavirus HKU8, Myotis ricketti alphacoronavirus Sax-2011, Nyctalus velutinus alphacoronavirus SC-2013, Porcine epidemic diarrhea virus, Scotophilus bat coronavirus 512, Rhinolophus bat coronavirus HKU2, Human coronavirus NL63, NL63-related bat coronavirus strain BtKYNL63-9b, Alphacoronavirus 1.*

As used herein, the term *"Betacoronavirus"* refers to the corresponding family of the Baltimore classification, which includes the *Embecovirus, Hibecovirus, Merbecovirus, Nobecovirus,* and *Sarbecovirus* subgenus. In a non-exhaustive manner, this includes the following species: *Betacoronavirus 1, China Rattus coronavirus HKU24, Human coronavirus HKU1, Murine coronavirus, Bat Hp-betacoronavirus Zhejiang2013, Hedgehog coronavirus 1, Middle East respiratory syndrome-related coronavirus, Pipistrellus bat coronavirus HKU5, Tylonycteris bat coronavirus HKU4, Hedgehog coronavirus 1, Middle East respiratory syndrome-related coronavirus, Pipistrellus bat coronavirus HKU5, Tylonycteris bat coronavirus HKU4, Rousettus bat coronavirus GCCDC1, Rousettus bat coronavirus HKU9, Severe acute respiratory syndrome-related coronavirus.*

As used herein, the term *"Severe acute respiratory syndrome-related coronavirus",* or SARS virus, includes, in a non-exhaustive manner, the *SARS-CoV, SARSr-CoV WIV1, SARSr-CoV HKU3, SARSr-CoV RP3,* and *SARS-CoV-2; including strains responsible for COVID-19 and their mutants.*

As used herein, the term *"Deltacoronavirus"* refers to the corresponding family of the Baltimore classification, which includes the *Andecovirus, Buldecovirus, Herdecovirus,* and *Moordecovirus* subgenus. In a non-exhaustive manner, this includes the following species: *Wigeon coronavirus HKU20, Bulbul coronavirus HKU11, Coronavirus HKU15, Munia coronavirus HKU13, White-eye coronavirus HKU16, Night heron coronavirus HKU19, Common moorhen coronavirus HKU21.*

As used herein, the term *"Gammacoronavirus"* refers to the corresponding family of the Baltimore classification, which includes the *Cegacovirus* and *Igacovirus* subgenus. In a non-exhaustive manner, this includes the following species: *Beluga whale coronavirus SW1* and *Avian coronavirus.*

Among single-stranded negatively RNA viruses of medical interest, one may cite measles virus, *Filoviridae* virus family encompassing the Ebola virus, the *Paramyxoviridae* family encompassing the Respiratory Syncytial virus (RSV), the *Rhabdoviridae* family, the *Orthomyxoviridae* family encompassing the Influenzavirus A, Influenzavirus B and Influenzavirus C.

Viruses of the *Mononegavirales* order are also particularly considered by the invention. The order *Mononegavirales* includes viruses belonging to Group V of the Baltimore classification. As of 2018, this order includes mainly the following virus families: *Bornaviridae, Mymonaviridae, Filoviridae, Nyamiviridae, Paramyxoviridae, Pneumoviridae, Rhabdoviridae,* and *Sunviridae.*

Human respiratory syncytial virus (HRSV) is a syncytial virus that causes respiratory tract infections. It is a major cause of lower respiratory tract infections and hospital visits during infancy and childhood. HRSV virus may in particular be considered by the invention and pertain to the Group V of RNA viruses. More particularly, RSV virus is a (-)ssRNA virus belonging to group V of the Baltimore classification. It is a pneumovirus which is part of the *Paramyxoviridae* family, which belongs to the *Mononegavirales* order. Among other viruses of the *Mononegavirales* order, those which are particularly considered by the invention include: measles virus, mumps virus, Nipah virus, rabies virus, and human parainfluenza virus (which includes HPIV-1, HPIV-2, HPIV-3 and HPIV-4). Of note, the *Paramyxovirinae* subfamily was conventionally merged into the *Paramyxoviridae* family, by reference to the taxonomy of the *Mononegavirales* order updated in 2016.

The virus genus which are particularly considered within the *Paramyxoviridae* family include: *Aquaparamyxovirus, Avulavirus, Ferlavirus, Henipavirus, Morbillivirus, Respirovirus* and *Rubulavirus* genus.

Viruses of the *Orthomyxoviridae* family are also particularly considered by the invention. The *Orthomyxoviridae* family belongs to an "Unassigned" order according to the 2017 Virus Taxonomy. The virus genus which are particularly considered within the *Orthomyxoviridae* family include: *Alphainfluenzavirus, Betainfluenzavirus, Deltainfluenzavirus, Gammainfluenzavirus, Isavirus, Quaranjavirus,* and *Thogotovirus.*

Influenzavirus A, Influenzavirus B, Influenzavirus C may in particular be considered by the invention and pertain to the Group V RNA viruses and the *Orthomyxoviridae* family, which can be defined as a negative-sense single-stranded RNA or (-)ss RNA viruses. Isavirus and Thogotovirus also belong to the *Orthomyxoviridae* order.

## Administration of the compounds of formula (I)

The compounds of formula (I) as defined here-above may be formulated into a pharmaceutical composition, in particular into a medicament, suitable for administration to a patient.

The present invention thus further relates to a medicament comprising the compound of formula (I).

The present invention thus further relates to a pharmaceutical composition, characterized in that it comprises the compound of formula (I), and at least one pharmaceutically acceptable excipient

The present invention also relates to a pharmaceutical composition comprising the compound of formula (I), and at least one pharmaceutically acceptable excipient, for use in the treatment and/or prevention of cancer and of virus infections, in particular RNA virus infections, such as RNA virus infections from group IV or V, as detailed herein above, and more particularly infections caused by Zika (ZIKV), Dengue (DENV), West Nile virus (WNV), Japanese encephalitis, Tick-borne encephalitis, Chikungunya (CHIKV), Ross River (RRV), Mayaro, measles virus or SARS-CoV-2.

In particular, the invention relates to a medicament comprising the compound of formula (I), for use in the treatment and/or prevention of cancer and of virus infections, in particular RNA virus infections, such as RNA virus infections from group IV or V, as detailed herein above, and more particularly infections caused by Zika (ZIKV), Dengue (DENV), West Nile virus (WNV), Japanese encephalitis, Tick-borne encephalitis, Chikungunya (CHIKV), Ross River (RRV), Mayaro, measles virus or SARS-CoV-2.

Alternatively, the invention relates to the use of the compound of formula (I) as defined above, for the preparation of a pharmaceutical composition or of a medicament for the treatment and/or prevention of cancer and of virus infections, in particular RNA virus infections, such as RNA virus infections from group IV or V, as detailed herein above, and more particularly infections caused by Zika (ZIKV), Dengue (DENV), West Nile virus (WNV), Japanese encephalitis, Tick-borne encephalitis, Chikungunya (CHIKV), Ross River (RRV), Mayaro, measles virus or SARS-CoV-2.

According to another aspect, herein is provided a method of treating the pathological conditions indicated above, and more particularly infections caused by Zika (ZIKV), Dengue (DENV), West Nile virus (WNV), Japanese encephalitis, Tick-borne encephalitis, Chikungunya (CHIKV), Ross River (RRV), Mayaro, measles virus or SARS-CoV-2, comprising administering to a subject in need thereof a therapeutically effective amount of the compound of formula (I). In an embodiment of this method of treatment, the subject is a human.

The pharmaceutically acceptable excipient is more particularly chosen among pharmaceutically carrier, adjuvant, or vehicle.

The choice of the acceptable excipient(s) to be used in combination with the compound of formula (I) as defined herein is part of the general knowledge of one having ordinary skills in the formulation of pharmaceutical compositions, in particular of medicaments.

Such excipients are well known to those skilled in the art and are described notably in "Ullmann's Encyclopedia of Industrial Chemistry 6th Ed." (various editors, 1989-1998, Marcel Dekker) and in "Pharmaceutical Dosage Forms and Drug Delivery Systems" (ANSEL et al, 1994, WILLIAMS & WILKINS).

The aforementioned excipients are selected according to the dosage form and the desired mode of administration.

Compositions of this invention may be administered in any manner, including, but not limited to, orally, parenterally, sublingually, transdermally, vaginally, rectally, transmucosally, topically, intranasally via inhalation, via buccal or intranasal administration, ophtalmologically or *via* an implanted reservoir, or combinations thereof. Parenteral administration includes, but is not limited to, intravenous, intra-arterial, intraperitoneal, subcutaneous, intramuscular, intra-thecal, intra-articular, intra-synovial, intrasternal, intrahepatic, intralesional, intra-tracheal, and intracranial injection or infusion techniques. The compositions of this invention may also be administered in the form of an implant, which allows slow release of the compositions as well as a slow controlled i.v. infusion.

For example, the compounds of formula (I) can be present in any pharmaceutical form which is suitable for enteral or parenteral administration, in association with appropriate excipients, for example in the form of plain or coated tablets, hard gelatin, soft shell capsules and other capsules, suppositories, or drinkable, such as suspensions, syrups, or injectable solutions or suspensions.

Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent. Among the acceptable vehicles and solvents that may be employed are water, Ringer's solution and isotonic sodium chloride solution. In addition, sterile, fixed oils are conventionally employed as a solvent or suspending medium.

Orally acceptable dosage forms include, but are not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined

with emulsifying and suspending agents. If desired, certain sweetening, flavoring or coloring agents may also be added.

Alternatively, pharmaceutically acceptable compositions of this invention may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols.

Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

In a particular embodiment, the compound of formula (I) according to the invention is administered orally or intravenously.

Such pharmaceutically acceptable compositions may also be considered in combination with other active compounds, or alternatively may include the compounds according to the invention in combination with other active agents.

The amount of compound of the present invention that may be combined with the excipient(s) to produce a composition in a single dosage form will vary depending upon the host treated and the particular mode of administration.

In the pharmaceutical compositions for oral, sublingual, subcutaneous, intramuscular, intravenous, topical, local, intra-tracheal, intranasal, transdermal or rectal administration, the compound of formula (I) above, may be administered in a unit administration form, in a mixture with conventional pharmaceutical excipients, to animals and to human beings for the treatment of the above disorders or diseases.

The unit administration forms appropriate include oral forms such as tablets, soft or hard gel capsules, powders, granules and oral solutions or suspensions, sublingual, buccal, intra-tracheal, intra-ocular and intra-nasal administration forms, forms for inhalative, topical, transdermal, subcutaneous, intra-muscular or intravenous administration, rectal administration forms and implants. For topical application it is possible to use the compound of formula (I) in creams, gels, ointments or lotions.

There may be particular cases in which higher or lower dosages are appropriate. According to usual practice, the dosage that is appropriate for each patient is determined by the doctor according to the mode of administration and the weight and response of the said patient.

It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of a compound of the present invention in the composition will also depend upon the particular compound in the composition.

Throughout the description, including the claims, the expression "comprising a" should be understood as being synonymous with "comprising at least one" unless specifically stated otherwise.

The expressions "between... and ..." and "ranging from ... to ..." should be understood to mean that the limits are inclusive, unless specified otherwise.

The following examples are provided as illustrations and in no way limit the scope of this invention.

## EXAMPLES

**Example 1:** Preparation of the compounds of formula (I)

### Plant material

Bark of *Comptonella drupacea* was collected in May 2008 in « Réserve spéciale de faune du col d'Amieu et Table Unio », South Province, New Caledonia. The corresponding voucher specimen POU-0234 has been deposited at the Herbarium of the IRD Center, Nouméa, New Caledonia. Bark was dried at a temperature not exceeding 40 °C using a hot air generator before being ground with a Foreplex F1 hammer mill and stored at room temperature.

### Extraction and isolation procedures

Ground bark (0.24 kg) of *Comptonella drupacea* was extracted with EtOAc (4 X 0.5 L, 1 h each, 35 °C) to yield 4.0 g of crude extract after concentration *in vacuo.* The residue was subjected to a 220 g silica gel column flash chromatography using a gradient of n-heptane/EtOAc/MeOH of increasing polarities (100:0:0 to 0:100:0 in 16 CV to 0:70:30 in 8 CV, 80 $mL.min^{-1}$) to afford 12 fractions, F1-F12, according to their Thin Layer Chromatography (TLC) profiles. F11 (750 mg) was purified by preparative HPLC (Nucleodur $C_{18}$, 10 mM ammonium acetate buffer/$CH_3CN$, 90:10 to 10:90 at 21 mL $min^{-1}$) to yield 3 subfractions of which subfraction 3 contained **C1** (8.0 mg, $t_R$ 30.5 min). Cleared of **C1**, subfraction 3 (119 mg) was further purified (preparative HPLC, Nucleodur $C_{18}$, 10 mM ammonium acetate buffer/$CH_3CN$, 50:50 to 30:70 at 21 mL $min^{-1}$) to afford **C2** (2.9 mg, $t_R$ 15.8 min) and **C3** (2.7 mg, $t_R$ 25.3 min). F12 (278 mg) was subjected to

preparative HPLC (Nucleodur $C_{18}$, 10 mM ammonium acetate buffer/$CH_3CN$, 50:50 to 30:70 at 21 mL $min^{-1}$) to yield **C4** (5.3 mg, $t_R$ 15.4 min), **C5** (1.7 mg, $t_R$ 17.5 min), **C6** (2.5 mg, $t_R$ 26.5 min) and **C7** (0.7 mg, $t_R$ 29.7 min).

Structure characterization

**C1** as defined here-above was obtained as a yellow amorphous solid. Its HRESIMS data showed a $[M + H]^+$ ion peak at *m/z* 750.5121 (calcd for $C_{38}H_{68}N_7O_8^+$ 750.5124) corresponding to the molecular formula $C_{38}H_{67}N_7O_8$ and indicating nine degrees of unsaturation. The $^1H$ NMR spectrum of **C1** as defined here-above in $CD_3CN$ showed three amide NH doublets at $\delta_H$ 7.48, 7.39, 6.99, seven $\alpha$-amino proton signals in the region of $\delta_H$ 4.02-5.45, three olefinic protons ($\delta_H$ 4.93, 5.02, 5.69), four N-methyl singlets ($\delta_H$ 2.86, 2.94, 2.96, 3.05) and ten methyl signals ($\delta_H$ 0.85-1.26) **(Table 1** below).

**Table 1.** $^1H$ NMR (700 MHz) and $^{13}C$ NMR (175 MHz) data of **C1-C2** in $CD_3CN$.

| | **C1** | | | **C2** | |
|---|---|---|---|---|---|
| position | $\delta_C$, type | $\delta_H$, mult. (*J* in Hz) | position | $\delta_C$, type | $\delta_H$, mult. (*J* in Hz) |
| **Isoleucine[1]** | | | **Isoleucine[1]** | | |
| NH | | 6.99, d (5.8) | NH | | 7.06, d (5.8) |
| CO | 173.7, C | | CO | 175.7, C | |
| $\alpha$ | 56.2, CH | 4.65, dd (5.8, 3.5) | $\alpha$ | 56.9, CH | 4.44, dd (7.9, 5.8) |
| $\beta$ | 34.6, CH | 1.85, m | $\beta$ | 36.9, CH | 1.77[a], m |
| $\gamma$a | 27.3, $CH_2$ | 1.40, m | $\gamma$a | 26.1, $CH_2$ | 1.18, m |
| $\gamma$b | | 1.50, m | $\gamma$b | | 1.44, m |
| $\gamma'$ | 14.1, $CH_3$ | 0.91, d (7.0) | $\gamma'$ | 15.7, $CH_3$ | 1.02, d (7.0) |
| $\delta$ | 12.1, $CH_3$ | 0.937, t (7.5) | $\delta$ | 12.0, $CH_3$ | 0.91, t (7.5) |
| ***N*-methyl-a lanine[2]** | | | ***N*-methyl-alanine[2]** | | |
| N-Me | 30.5, $CH_3$ | 2.96, s | N-Me | 31.7, $CH_3$ | 3.08, s |
| CO | 170.0, C | | CO | 171.3, C | |
| a | 50.7, CH | 5.45, q (6.5) | a | 52.3, CH | 5.15, q (7.0) |
| $\beta$ | 15.8, $CH_3$ | 1.15, d (6.5) | $\beta$ | 13.1, $CH_3$ | 1.21, d (7.0) |
| ***N*-methyl-l eucine[3]** | | | **Leucine[3]** | | |
| N-Me | 29.9, $CH_3$ | 2.86, s | NH | | 7.16, d (7.2) |
| CO | 169.6, C | | CO | 172.3, C | |
| $\alpha$ | 59.7, CH | 4.02, dd (11.2, 3.9) | $\alpha$ | 54.2, CH | 3.55, m |
| $\beta$a | 40.2, $CH_2$ | 1.15[a], m | $\beta$a | 37.1, $CH_2$ | 1.71, m |
| $\beta$b | | 2.05, m | $\beta$b | | 1.89, m |
| $\gamma$ | 25.9, CH | 1.35, m | $\gamma$ | 25.7, CH | 1.55, m |
| $\delta$ | 23.9, $CH_3$ | 0.85, d (6.7) | $\delta$ | 23.7, $CH_3$ | 0.88, d (6.7) |
| $\delta'$ | 22.8, $CH_3$ | 0.935, d (6.7) | $\delta'$ | 21.5 $CH_3$ | 0.83 d (6.7) |
| **2-amino-4-methyl-5-hexenoic acid[4]** | | | **2-amino-4-methyl-5-hexenoic acid[4]** | | |
| NH | | 7.48, d (8.6) | NH | | 7.00, d (9.0) |
| CO | 174.7, C | | CO | 176.0, C | |
| $\alpha$ | 50.3, CH | 4.74, ddd (12.0, 8.6, 2.0) | $\alpha$ | 50.4, CH | 4.76[a], m |
| $\beta$a | 36.7, $CH_2$ | 1.69, ddd (14.7, 12.0, 3.2) | $\beta$a | 36.7, $CH_2$ | 1.77[a], m |
| $\beta$b | | 1.54, ddd (14.7, 11.0, 2.0) | $\beta$b | | 1.49, ddd (14.7, 11.5, 1.7) |
| $\gamma$ | 36.8, CH | 2.14[a], m | $\gamma$ | 36.1, CH | 2.24, m |
| $\delta$ | 143.6, CH | 5.69, ddd (8.9, 10.0, 17.0) | $\delta$ | 143.1, CH | 5.67, m |
| $\delta'$ | 21.4, $CH_3$ | 0.98, d (7.0) | $\delta'$ | 21.1, $CH_3$ | 0.98, d (6.8) |
| $\varepsilon$a | 115.7, $CH_2$ | 4.93, dd (17.0, 1.5) | $\varepsilon$a | 116.8, $CH_2$ | 5.07, brs |
| $\varepsilon$b | | 5.02, dd (10.0, 1.5) | $\varepsilon$b | | 5.09, m |

(continued)

| | | | | | |
|---|---|---|---|---|---|
| ***N*-methyl-alanine[5]** | | | ***N*-methyl-alanine[5]** | | |
| N-Me | 31.9, $CH_3$ | 3.05, s | N-Me | 32.1, $CH_3$ | 3.04, s |
| CO | 175.9, C | | CO | 175.8, C | |
| a | 52.4, CH | 4.84, q (7.3) | a | 52.9, CH | 4.77 [a], m |
| $\beta$ | 13.7, $CH_3$ | 1.33, d (7.3) | $\beta$ | 13.8, $CH_3$ | 1.32, d (7.3) |
| ***N*-methyl-alanine[6]** | | | ***N*-methyl-** | **alanine[6]** | |
| N-Me | 31.2, $CH_3$ | 2.94, s | N-Me | 31.0, $CH_3$ | 2.91, s |
| CO | 170.5, C | | CO | 170.3, C | |
| $\alpha$ | 52.6, CH | 5.21, q (7.2) | $\alpha$ | 52.4, CH | 5.22, q (7.2) |
| $\beta$ | 14.3, $CH_3$ | 1.26, d (7.2) | $\beta$ | 13.8, $CH_3$ | 1.24, d (7.2) |
| **3-hydroxy-leucine[7]** | | | **3-hydroxy-leucine[7]** | | |
| NH | | 7.39, d (9.5) | NH | | 7.43, d (9.5) |
| CO | 174.3, C | | CO | 172.8, C | |
| $\alpha$ | 55.9, CH | 4.70, t (9.5) | $\alpha$ | 55.4, CH | 4.70, dd (9.5, 5.5) |
| $\beta$ | 76.8, CH | 3.48, d (9.5) | $\beta$ | 80.1, CH | 3.50, t (5.5) |
| $\beta$-OH | | 5.15, brs | $\beta$-OH | | 4.58, brs |
| $\gamma$ | 29.3, CH | 1.78, h (7.0) | $\gamma$ | 30.1, CH | 1.98, m |
| $\delta$ | 14.8, $CH_3$ | 0.927[a], m | $\delta$ | 17.3, $CH_3$ | 0.92, d (6.6) |
| $\delta'$ | 21.2, $CH_3$ | 0.97, d (7.0) | $\delta'$ | 20.5, $CH_3$ | 1.01, d (6.6) |

[a]Overlapped by other signals

**Table 2.** $^1H$ NMR (700 MHz) and $^{13}C$ NMR (175 MHz) data of **C3-C4** in $CD_3CN$.

| | C3 | | | C4 | |
|---|---|---|---|---|---|
| position | $\delta_C$, type | $\delta_H$, mult. ($J$ in Hz) | position | $\delta_C$, type | $\delta_H$, mult. ($J$ in Hz) |
| **Isoleucine[1]** | | | **Isoleucine[1]** | | |
| NH | | 7.01, d (5.8) | NH | | 7.10, d (5.8) |
| CO | 173.7, C | | CO | 173.7, C | |
| $\alpha$ | 56.2, CH | 4.65, dd (5.8, 3.5) | $\alpha$ | 56.3, CH | 4.65, dd (6.0, 3.5) |
| $\beta$ | 34.6, CH | 1.85, m | $\beta$ | 34.6, CH | 1.86, m |
| $\gamma$a | 27.3, $CH_2$ | 1.40, m | $\gamma$a | 27.3, $CH_2$ | 1.40, m |
| $\gamma$b | | 1.50, m | $\gamma$b | | 1.50, m |
| $\gamma'$ | 14.1, $CH_3$ | 0.91, d (7.0) | $\gamma'$ | 14.1, $CH_3$ | 0.91, d (7.0) |
| $\delta$ | 12.1, $CH_3$ | 0.94 [a], m | $\delta$ | 12.1, $CH_3$ | 0.93[a],m |
| ***N*-methyl-alanine[2]** | | | ***N*-methyl-alanine[2]** | | |
| N-Me | 30.5, $CH_3$ | 2.97, s | N-Me | 30.5, $CH_3$ | 2.97, s |
| CO | 170.0, C | | CO | 170.0, C | |
| *a* | 50.7, CH | 5.46, q (6.5) | *a* | 50.8, CH | 5.46, q (6.5) |
| $\beta$ | 15.8, $CH_3$ | 1.16, d (6.5) | $\beta$ | 15.8, $CH_3$ | 1.16, d (6.5) |
| ***N*-methyl-homoisoleucine[3]** | | | ***N*-methyl-5-hydroxy-leucine[3]** | | |
| N-Me | 29.9, $CH_3$ | 2.87, s | N-Me | 29.9, $CH_3$ | 2.88, s |
| CO | 169.5, C | | CO | 169.7, C | |
| *a* | 59.5, CH | 4.05, dd (11.2, 3.9) | *a* | 59.5, CH | 4.08, dd (11.5, 3.7) |
| $\beta$a | 38.2, $CH_2$ | 1.07, m | $\beta$a | 34.7, $CH_2$ | 1.07, ddd (13.2, 9.5, 3.7) |
| $\beta$b | | 2.13, m | $\beta$b | | 2.23, ddd (13.2, 11.5, 4.0) |

(continued)

| | | | | | |
|---|---|---|---|---|---|
| ***N*-methyl-homoisoleucine[3]** | | | ***N*-methyl-5-hydroxy-leucine[3]** | | |
| $\gamma$ | 31.8, CH | 1.08, m | $\gamma$ | 33.5, CH | 1.32[a], m |
| $\delta$a | 31.2, $CH_2$ | 1.15 [a], m | $\delta$ | 67.8, $CH_2$ | 3.25, m |
| $\delta$b | | 1.29, m | $\delta'$ | 17.3, $CH_3$ | 0.925[a], m |
| $\delta'$ | 19.6, CH3 | 0.93 [a], m | $\delta$-OH | | |
| $\varepsilon$ | 11.6, $CH_3$ | 0.82, t (7.3) | | | |
| **2-amino-4-methyl-5-hexenoic acid[4]** | | | **2-amino-4-methyl-5-hexenoic** acid[4] | | |
| NH | | 7.49, d (8.6) | NH | | 7.56, d (8.7) |
| CO | 174.7, C | | CO | 174.7, C | |
| $\alpha$ | 50.3, CH | 4.75, ddd (12.0, 8.6, 2.0) | $\alpha$ | 50.4, CH | 4.75, ddd (12.0, 8.6, 2.0) |
| $\beta$a | 36.7, $CH_2$ | 1.70, ddd (14.7, 12.0, 3.2) | $\beta$a | 36.7, $CH_2$ | 1.69, ddd (14.7, 12.0, 3.2) |
| $\beta$b | | 1.54, ddd (14.7, 11.0, 2.0) | $\beta$b | | 1.56, ddd (14.7, 11.0, 2.0) |
| $\gamma$ | 36.8, CH | 2.14[a], m | $\gamma$ | 36.8, CH | 2.14[a], m |
| $\delta$ | 143.6, CH | 5.69, ddd (8.9, 10.0, 17.0) | $\delta$ | 143.5, CH | 5.70, ddd (8.9, 10.0, 17.0) |
| $\delta'$ | 21.4, $CH_3$ | 0.99, d (7.0) | $\delta'$ | 21.4, $CH_3$ | 0.99, d (7.0) |
| $\varepsilon$a | 115.7, $CH_2$ | 4.94, dd (17.0, 1.5) | $\varepsilon$a | 115.8, $CH_2$ | 4.94, dd (17.0, 1.5) |
| $\varepsilon$b | | 5.02, dd (10.0, 1.5) | $\varepsilon$b | | 5.02, dd (10.0, 1.5) |
| ***N*-methyl-alanine[5]** | | | ***N*-methyl-alanine[5]** | | |
| N-Me | 31.9, $CH_3$ | 3.06, s | N-Me | 31.9, $CH_3$ | 3.06, s |
| CO | 175.9, C | | CO | 175.9, C | |
| $\alpha$ | 52.3, CH | 4.86, q (7.3) | $\alpha$ | 52.4, CH | 4.86, q (7.3) |
| $\beta$ | 13.7, $CH_3$ | 1.34, d (7.3) | $\beta$ | 13.7, $CH_3$ | 1.34, d (7.3) |
| ***N*-methyl-alanine[6]** | | | ***N*-methyl-alanine[6]** | | |
| N-Me | 31.2, $CH_3$ | 2.95, s | N-Me | 31.2, $CH_3$ | 2.95, s |
| CO | 170.5, C | | CO | 170.6, C | |
| $\alpha$ | 52.6, CH | 5.22, q (7.2) | $\alpha$ | 52.7, CH | 5.22, q (7.2) |
| $\beta$ | 14.3, $CH_3$ | 1.27, d (7.2) | $\beta$ | 14.3, $CH_3$ | 1.26, d (7.2) |
| **3-hydroxy-leucine[7]** | | | **3-hydroxy-leucine[7]** | | |
| NH | | 7.39, d (9.5) | NH | | 7.38, d (9.5) |
| CO | 174.3, C | | CO | 174.2, C | |
| $\alpha$ | 55.8, CH | 4.71, t (9.5) | $\alpha$ | 55.8, CH | 4.72, t (9.5) |
| $\beta$ | 76.8, CH | 3.47, dd (9.5, 1.5) | $\beta$ | 76.8, CH | 3.48, dd (1.3, 9.5) |
| $\beta$-OH | | 5.14, brs | $\beta$-OH | | - |
| $\gamma$ | 29.3, CH | 1.79, m | $\gamma$ | 29.3, CH | 1.78, h (7.0) |
| $\delta$ | 14.8, $CH_3$ | 0.93[a], m | $\delta$ | 14.9, $CH_3$ | 0.92[a], m |
| $\delta'$ | 21.2, $CH_3$ | 0.98, d (7.0) | $\delta'$ | 21.2, $CH_3$ | 0.98, d (7.0) |

"Overlapped by other signals

**Table 3.** $^1$H NMR (700 MHz) and $^{13}$C NMR (175 MHz) data of **C5-C6** in $CD_3CN$.

| | C5 | | | C6 | |
|---|---|---|---|---|---|
| position | $\delta_C$, type | $\delta_H$, mult; (*J* in Hz) | position | $\delta_C$, type | $\delta_H$, mult. (Jin Hz) |
| **Isoleucine[1]** | | | **Isoleucine[1]** | | |
| NH | | 7.04, d (5.8) | NH | | 7.05, d (5.8) |
| CO | 173.6, C | | CO | 173.7, C | |

(continued)

| | C5 | | C6 | | |
|---|---|---|---|---|---|
| position | $\delta_C$, type | $\delta_H$, mult; (*J* in Hz) | position | $\delta_C$, type | $\delta_H$, mult. (Jin Hz) |
| **Isoleucine[1]** | | | **Isoleucine[1]** | | |
| $\alpha$ | 56.2, CH | 4.65, dd (5.8, 3.5) | $\alpha$ | 56.3, CH | 4.65, dd (6.0, 3.5) |
| $\beta$ | 34.6, CH | 1.86, m | $\beta$ | 34.5, CH | 1.86, m |
| $\gamma$a | 27.3, $CH_2$ | 1.40, m | $\gamma$a | 27.3, $CH_2$ | 1.41, m |
| $\gamma$b | | 1.50, m | $\gamma$b | | 1.51, m |
| $\gamma'$ | 14.1, $CH_3$ | 0.91, d (7.0) | y' | 14.1, $CH_3$ | 0.91, d (7.0) |
| $\delta$ | 12.1, $CH_3$ | 0.94[a], m | $\delta$ | 12.1, $CH_3$ | 0.94[a],m |
| ***N*-methyl-alanine[2]** | | | ***N*-methyl-alanine[2]** | | |
| N-Me | 30.5, $CH_3$ | 2.97, s | N-Me | 30.5, $CH_3$ | 2.97, s |
| CO | 170.0, C | | CO | 170.0, C | |
| $\alpha$ | 50.8, CH | 5.45, q (6.5) | $\alpha$ | 50.7, CH | 5.46, q (6.5) |
| $\beta$ | 15.8, $CH_3$ | 1.16, d (6.5) | $\beta$ | 15.8, $CH_3$ | 1.16, d (6.5) |
| ***N*-methyl-5-hydroxy-homoisoleucine[3]** | | | ***N*-methyl-5-hydroxyformyl-leucine[3]** | | |
| N-Me | 29.9, $CH_3$ | 2.88, s | N-Me | 29.8, $CH_3$ | 2.87, s |
| CO | 169.6, C | | CO | 169.1, C | |
| $\alpha$ | 59.7, CH | 4.06, dd (12.0, 3.7) | $\alpha$ | 59.1, CH | 4.08, dd (11.5, 3.7) |
| $\beta$a | 34.5, $CH_2$ | 1.10, ddd (13.2, 9.5, 3.7) | $\beta$a | 34.6, $CH_2$ | 1.16, m |
| $\beta$b | | 2.25, ddd (13.2, 12.0, 2.8) | $\beta$b | | 2.24, m |
| $\gamma$ | 37.1, CH | 1.17[a], m | $\gamma$ | 30.3, CH | 1.56, m |
| $\delta$ | 71.4, CH | 3.50, dq (6.3, 4.3) | $\delta$ | 69.3, $CH_2$ | 3.90, m |
| $\delta'$ $\delta$-OH | 14.9, $CH_3$ | 0.92[a], m | $\delta'$ CHO | 16.9, $CH_3$ 162.4, CH | 1.00[a], m 8.03, s |
| $\varepsilon$ | 20.1, $CH_3$ | 1.00[a], m | | | |
| **2-amino-4-methyl-5-hexenoic acid[4]** | | | **2-amino-4-methyl-5-hexenoic** acid[4] | | |
| NH | | 7.53, d (8.6) | NH | | 7.53, d (8.7) |
| CO | 174.6, C | | CO | 174.6, C | |
| $\alpha$ | 50.4, CH | 4.74, ddd (12.0, 8.6, 2.0) | $\alpha$ | 50.4, CH | 4.74, m |
| $\beta$a | 36.6, $CH_2$ | 1.69, ddd (14.7, 12.0, 3.2) | $\beta$a | 36.5, $CH_2$ | 1.69, ddd (14.7, 12.0, 3.2) |
| $\beta$b | | 1.55, ddd (14.7, 11.0, 2.0) | $\beta$b | | 1.56, m |
| $\gamma$ | 36.8, CH | 2.14[a], m | $\gamma$ | 36.8, CH | 2.14[a], m |
| $\delta$ | 143.5, CH | 5.69, ddd (8.9, 10.0, 17.0) | $\delta$ | 143.5, CH | 5.69, m |
| $\delta'$ | 21.4, $CH_3$ | 0.99, d (7.0) | $\delta'$ | 21.4, $CH_3$ | 0.99, d (7.0) |
| $\varepsilon$a | 115.7, $CH_2$ | 4.94, dd (17.0, 1.5) | $\varepsilon$a | 115.7, $CH_2$ | 4.93, dd (17.0, 1.7) |
| $\varepsilon$b | | 5.02, dd (10.0, 1.5) | $\varepsilon$b | | 5.02, dd (10.0, 1.7) |
| ***N*-methyl-alanine[5]** | | | ***N*-methyl-alanine[5]** | | |
| N-Me | 31.9, $CH_3$ | 3.06, s | N-Me | 31.9, $CH_3$ | 3.06, s |
| CO | 175.9, C | | CO | 175.9, C | |
| $\alpha$ | 52.4, CH | 4.87, q (7.3) | $\alpha$ | 52.3, CH | 4.86, q (7.3) |

| | | | | | |
|---|---|---|---|---|---|
| β | 13.7, $CH_3$ | 1.34, d (7.3) | β | 13.7, $CH_3$ | 1.34, d (7.3) |

(continued)

| | | | | | |
|---|---|---|---|---|---|
| ***N*-methyl-alanine[6]** | | | ***N*-methyl-alanine[6]** | | |
| N-Me | 31.2, $CH_3$ | 2.95, s | N-Me | 31.2, $CH_3$ | 2.95, s |
| CO | 170.6, C | | CO | 170.6, C | |
| $\alpha$ | 52.6, CH | 5.22, q (7.2) | $\alpha$ | 52.7, CH | 5.22, q (7.2) |
| $\beta$ | 14.3, $CH_3$ | 1.27, d (7.2) | $\beta$ | 14.3, $CH_3$ | 1.26, d (7.2) |
| **3-hydroxy-leucine[7]** | | | **3-hydroxy-leucine[7]** | | |
| NH | | 7.37, d (9.5) | NH | | 7.37, d (9.5) |
| CO | 174.3, C | | CO | 174.2, C | |
| $\alpha$ | 55.8, CH | 4.71, t (9.5) | $\alpha$ | 55.8, CH | 4.72, t (9.5) |
| $\beta$ | 76.8, CH | 3.48, d (9.5) | $\beta$ | 76.8, CH | 3.45, d (9.5) |
| $\beta$-OH | | 5.14, brs | $\beta$-OH | | 5.16, brs |
| $\gamma$ | 29.4, CH | 1.78, h (7.0) | $\gamma$ | 29.3, CH | 1.78, m |
| $\delta$ | 14.8, CH3 | 0.93[a], m | $\delta$ | 14.9, CH3 | 0.93[a], m |
| $\delta$' | 21.3, CH3 | 0.98[a], m | $\delta$' | 21.2, CH3 | 0.98, d (7.0) |

[a]Overlapped by other signals

**Table 4.** $^1H$ NMR (700 MHz) and $^{13}C$ NMR (175 MHz) data of **C7** in $CD_3CN$.

| | **C7** | |
|---|---|---|
| position | $\delta_C$, type | $\delta_H$, mult. ($J$ in Hz) |
| **Isoleucine[1]** | | |
| NH | | 7.03, d (5.8) |
| CO | 173.7, C | |
| $\alpha$ | 56.3, CH | 4.65, dd (5.8, 3.5) |
| $\beta$ | 34.5, CH | 1.86, m |
| $\gamma$a | 27.3, $CH_2$ | 1.41, m |
| $\gamma$b | | 1.51, m |
| $\gamma$' | 14.1, $CH_3$ | 0.91, d (7.0) |
| $\delta$ | 12.1, CH3 | 0.94[a], m |
| ***N*-methyl-alanine[2]** | | |
| N-Me | 30.5, $CH_3$ | 2.97, s |
| CO | 170.0, C | |
| a | 50.8, CH | 5.45, q (6.5) |
| $\beta$ | 15.8, $CH_3$ | 1.16, d (6.5) |
| ***N*-methyl-5-hydroxyformyl-homoisoleucine[3]** | | |
| N-Me | 29.8, $CH_3$ | 2.87, s |
| CO | 169.1, C | |
| $\alpha$ | 59.3, CH | 4.06, dd (12.0, 3.7) |
| $\beta$a | 34.1, $CH_2$ | 1.15[a], m |
| $\beta$b | | 2.23, m |
| $\gamma$ | 35.0, CH | 1.38[a], m |
| $\delta$ | 75.3, CH | 4.78, quint (6.3) |
| $\delta$' | 15.1, $CH_3$ | 0.99[a], m |
| $\varepsilon$ | 17.1, $CH_3$ | 1.14, d (6.5) |

(continued)

| | | |
|---|---|---|
| ***N*-methyl-5-hydroxyformyl-homoisoleucine**[3] | | |
| CHO | 162.1, CH | 8.02, s |
| **2-amino-4-methyl-5-hexenoic** acid[4] | | |
| NH | | 7.52, d (8.6) |
| CO | 174.6, C | |
| $\alpha$ | 50.4, CH | 4.74, m |
| $\beta$a | 36.6, $CH_2$ | 1.68, m |
| $\beta$b | | 1.55, ddd (14.7, 11.0, 2.0) |
| $\gamma$ | 36.8, CH | 2.14, m |
| $\delta$ | 143.5, CH | 5.69, m |
| $\delta$' | 21.4, $CH_3$ | 0.99, d (7.0) |
| $\varepsilon$a | 115.7, $CH_2$ | 4.94, dd (17.0, 1.5) |
| $\varepsilon$b | | 5.02, dd (10.0, 1.5) |
| ***N*-methyl-alanine**[5] | | |
| N-Me | 31.9, $CH_3$ | 3.06, s |
| CO | 175.9, C | |
| $\alpha$ | 52.3, CH | 4.87, q (7.3) |
| $\beta$ | 13.7, $CH_3$ | 1.34, d (7.3) |
| ***N*-methyl-alanine**[6] | | |
| N-Me | 31.2, $CH_3$ | 2.95, s |
| CO | 170.6, C | |
| $\alpha$ | 52.6, CH | 5.22, q (7.2) |
| $\beta$ | 14.3, $CH_3$ | 1.27, d (7.2) |
| **3-hydroxy-leucine**[7] | | |
| NH | | 7.35, d (9.5) |
| CO | 174.2, C | |
| $\alpha$ | 55.8, CH | 4.72, t (9.5) |
| $\beta$ | 76.8, CH | 3.45, d (9.5) |
| $\beta$-OH | | 5.15, brs |
| $\gamma$ | 29.3, CH | 1.78, h (7.0) |
| $\delta$ | 14.8, CH3 | 0.92[a], m |
| $\delta$' | 21.3, CH3 | 0.99 [a], m |
| [a]Overlapped by other signals | | |

The $^{13}C$ NMR spectrum contained seven amide-type carbonyl signals ($\delta_C$ 169.5-175.9), two vinylic carbons ($\delta_C$ 115.7, 143.6) and an oxygenated methine ($\delta_C$ 76.8) (**Table 1** above). The investigation of COSY, TOCSY, HSQC-TOCSY, HSQC, HMBC and ROESY spectra (**Figures 1-6**) allowed the identification of seven amino acids, one isoleucine (Ile), one N-methyl leucine [(NMe)Leu], one hydroxyleucine (OH-Leu), three N-methyl alanines [(NMe)Ala] and the unusual 2-amino-4-methyl-5-hexenoic acid (dehydro-methylleucine, dhMleu) residue. The TOCSY correlations from the methylene protons **H**-$\varepsilon$a and **H**-$\varepsilon$b to H-$\delta$', H-$\gamma$, $H_3$-$\delta$, H-$\beta$a, H-$\beta$b and H-$\alpha$, as well as the COSY correlation from H-$\alpha$ to NH highlighted the spin system of the dhMleu[4] fragment. The HMBC correlations from H-$\alpha$ to C-$\gamma$, H-$\beta$a and H-$\beta$b to C-$\delta$', $H_3$-$\delta$ to C-$\beta$ and C-$\delta$' and from H-$\varepsilon$a to C-$\gamma$ confirmed the structure of the residue. All features accounted for eight of nine degrees of unsaturation, suggesting the cyclic structure of **C1** as defined here-above.

The sequence of the amino acid residues was defined using HMBC correlations observed from each NH or *N*Me to its respective adjacent carbonyl. The correlations from Ile[1]-NH to OH-Leu[7]-CO, OH-Leu[7]-NH to (*N*Me)Ala[6]-CO, (*N*Me)Ala[6]-*N*Me to (*N*Me)Ala[5]-CO, (*N*Me)Ala[5]-*N*Me to dhMLeu[4]-CO, dhMLeu[4]-NH to (*N*Me)Leu[3]-CO, (*N*Me)Leu[3]-*N*Me to (*N*Me)Ala[2]-CO and (*N*Me)Ala[2]-*N*Me to Ile[1]-CO, indicated that **C1** as defined here-above was linked in a cyclo-sequence [Ileu[1], (NMe)Ala[2], (*N*Me)Leu[3], dhMleu[4], (*N*Me)Ala[5], (*N*Me)Ala[6], OH-Leu[7]].

Analysis of ESI-MS/MS fragmentation patterns in the positive mode indicated that cleavages occur predominantly at the C-terminal of *N*Me-Ala[2,5,6], as evidenced by the key MS/MS fragments at *m/z* 423 and 338, followed by the sequential losses of *N*Me-Ala[5] and dhMleu[4] (*m/z* 253 and 128) (**Figures 7-8**). Furthermore, the peaks at *m/z* 625, 498, 413, 300, 171 and 86 demonstrated the sequential cleavage of dhMleu[4], (*N*Me)Leu[3], (*N*Me)Ala[2], Ile[1], OH-Leu[7], (*N*Me)Ala[6] and (*N*Me)Ala[5] and confirmed the sequence of the heptapeptide. The structure of **C1** as defined here-above was closely related to the natural peptide ternatin (Shimokawa et al, (-)-Ternatin, a highly N-methylated cyclic heptapeptide that inhibits fat accumulation: structure and synthesis. Tetrahedron Letters, 47 (2006) 4445-4448). The difference was the presence of dhMleu[4] in **C1** as defined here-above instead of Leu in ternatin. The absolute configurations of the amino acid residues of **C1** as defined here-above were partially assigned by HPLC analysis of its acid hydrolysate derivatized with Marfey's reagent (1-fluoro-2,4-dinitrophenyl-5-L-alanine amid). Two L-(*N*Me)Ala, one D-(*N*Me)Ala and L-(*N*Me)Leu were identified by comparison with retention times of commercially available standards. The stereochemistry of Ile[1] was D- or D-*allo* form, while that of OH-Leu[7] was L- or D- form. The N-H proton signals of OH-Leu[7] and dhMleu[4] remained on the $^1$H NMR spectrum when a small amount of $D_2O$ was added to the NMR tube, suggesting the presence of two intramolecular hydrogen bonds fixing **C1** as defined here-above in a single conformation. This was also described for ternatin, with the difference that a third hydrogen bond implying the hydroxy group of OH-Leu was also mentioned. This was not proved for **C1** as defined here-above. However, the same coupling constant of 9.5 *Hz* between the methine protons H-$\alpha$ and H-$\beta$ of OH-Leu[7] was observed, confirming an *anti* arrangement. The specific rotation value ( $[\alpha]_D^{20}$ -33 , *c* 0.4, EtOH) of **C1** as defined here-above was also similar to that of ternatin. Based on ROESY correlations and comparison with data from ternatin, a plausible absolute stereochemistry of **C1** as defined here-above was D-*allo*-Ileu[1], L-(*N*Me)Ala[2], L-(*N*Me)Leu[3], L-(*N*Me)Ala[5] and D-(*N*Me)Ala[6]. Synthetic studies are being conducted to assign unambiguously the absolute stereostructures of OH-Leu[7] and dhMleu[4].

The molecular formula of **C2** as defined here-above was established as $C_{37}H_{65}N_7O_8$ by HRESIMS, which showed a $[M + H]^+$ ion peak at *m/z* 736.4971 (calcd for $C_{37}H_{66}N_7O_8^+$ 736.4967). NMR spectroscopic data showed the presence of similar structural features to those of **C1** (**Table 1**). However, **C2** was found to possess a Leu[3] instead of a (*N*Me)Leu[3] as in **C1**. This was supported by the presence of an additional NH doublet at $\delta_H$ 7.16 and one less *N*-methyl singlet in the $^1$H spectrum. The TOCSY correlations from Leu[3]-NH to H-$\alpha$, H-$\beta$a, H-$\beta$b, H-$\gamma$, H-$\delta$ and H-$\delta'$ revealed the complete spin system of Leu[3]. The HMBC correlations from Leu[3]-NH ($\delta_H$ 7.16) to (*N*Me)Ala[2]-CO ($\delta_C$ 171.3) and from dhMLeu[4]-NH ($\delta_H$ 7.00) to Leu[3]-CO ($\delta_C$ 172.3) confirmed the position of the residue. The presence of this NH in place of an *N*Me considerably affects the chemical shifts of atoms throughout the molecule. We assume that this effect results from a significant conformational difference between **C1** and **C2.**

**C3** as defined here-above gave the molecular formula $C_{39}H_{69}N_7O_8$, as indicated by HRESIMS of the $[M + H]^+$ ion peak at *m/z* 764.5279 (calcd for $C_{39}H_{70}N_7O_8^+$ 764.5280). The formula suggested the presence of an additional methylene in the structure compared to that of **C1**. Its NMR spectra also displayed very similar proton and carbon resonances and cross correlations with those of **C1** (**Table 2**). The minor structural difference was the presence of (*N*Me)HomoIleu[3] in **C3** instead of (*N*Me)Leu[3] as in **C1**. This was evidenced by the vicinal coupling between the methine hydrogen H-$\alpha$ ($\delta_H$ 4.05) and the methylene $H_2$-$\beta$ ($\delta_H$ 1.07, 2.13), between H-$\gamma$ ($\delta_H$ 1.08) and the methyl $H_3$-$\delta$ ($\delta_H$ 0.93) and between $H_3$-$\varepsilon$ ($\delta_H$ 0.82) and $H_2$-$\delta'$ ($\delta_H$ 1.15, 1.29), as well as HMBC correlations from H-$\alpha$ to C-$\beta$ and C-$\gamma$ and from $H_3$-$\varepsilon$ to C-$\delta'$ and C-$\gamma$.

**C4** as defined here-above contains an additional oxygen atom in comparison with **C1**, as evidenced by its molecular formula $C_{38}H_{67}N_7O_9$ indicated by HRESIMS of the $[M + H]^+$ ion peak at *m/z* 766.5106 (calcd for $C_{38}H_{68}N_7O_9^+$ 766.5073). The NMR spectroscopic data of **C4** (**Table 2**) were very comparable to those of **C1** and **C3,** but revealed deshielded signals at $\delta_H$ 3.25 (**$H_2$-$\delta'$**) and $\delta_C$ 67.8 (C-$\delta'$) suggesting the presence of an oxygenated methylene on the (*N*Me)-5-OH-Leu[3] amino acid residue. The vicinal couplings observed from the COSY spectrum together with key HMBC correlations confirmed the structure of the side chain.

The structure of **C5,** which has the formula $C_{39}H_{69}N_7O_9$, only differed from compound **C4** by the possession of an additional methyl group and an oxygenated methine resonating at $\delta_H$ 3.50 (**H-$\delta$a**) and $\delta_C$ 71.4 (C-$\delta$) in place of the methylene-$\delta'$ as in **C4** (See **Table 3**). This was deduced from the COSY correlation between the methyl **$H_3$-$\varepsilon$** ($\delta_H$ 1.00) and the methine $\delta_H$ 3.50 (H-$\delta$a) and from correlations from **$H_3$-$\varepsilon$** to **H-$\delta$a** ($\delta_H$ 3.50), H-$\gamma$ ($\delta_H$ 1.17), **H-$\delta$**($\delta_H$ 0.92) (TOCSY spectrum) and from $H_3$-$\varepsilon$ to C-$\delta'$ and C-$\gamma$(HMBC spectrum).

HRESIMS data of compound **C6** as defined here-above showed a $[M + H]^+$ ion peak at *m/z* 794.5013 (calcd for $C_{39}H_{68}N_7O_{10}^+$ 794.5022) corresponding to the molecular formula $C_{39}H_{67}N_7O_{10}$ and indicating ten degrees of unsaturation. The molecule contains an additional unsaturation compared to compounds **C1-C5,** due to the presence of a formate group at the end of the (*N*Me)-5-formate-Leu[3] side chain. This was supported by the presence of the deshielded signals at $\delta_H$ 8.03 (**H-$\varepsilon$**) and $\delta_C$ 162.4 (C-$\varepsilon$) and key correlations observed from 2D NMR spectra: the proton **H-$\varepsilon$** of the formate group gave an HMBC correlation with the methylene carbon C-$\delta'$, a COSY correlation with the methylene proton

**H**-$\delta$a and TOCSY correlations with **H**-$\delta$a, **H**-$\gamma$and the methyl-$\delta$. The presence of this formate group in place of a hydroxyl in **C4** is the only structural difference between **C4** and **C6.**

HRESIMS and NMR data analysis of compound **C7** ($[M + H]^+$ ion peak at *m/z* 808.5212 (calcd for $C_{40}H_{70}N_7O_{10}^+$ 808.5179)), suggested the same single structural difference between **C5** and **C7** as between **C4** and **C6** (**Table 4**). The (*N*Me)-5-formate-HomoILeu$^3$ side chain also bears a formate group instead of a hydroxyl as in **C5.**

**Example 2:** Biological activity

1. Antiviral activity against ZIKV, RRV, and DENV

Protocols:

The antiviral activity of the compounds (**C1** and **C3** to **C7**) of formula (I) has been evaluated against Zika virus as representative of flaviviruses. Vero, A549 or Huh7.5 cells were infected with ZIKV$^{GFP}$ (MOI=2) and simultaneously treated with increasing concentrations of the compounds of formula (I) (**C1** and **C3** to **C7**) or ternatin (ten-fold serial dilutions of considered compound). At 24 h post-infection, the percentage of GFP expressed by the cells was measured by FACS (Flow cytometric analysis of GFP fluorescence).

The compound **C1** has been tested on several other positive polarity single-stranded RNA viruses, such as Ross River virus and dengue virus as representatives of alphaviruses as follows.

Vero cells were infected with ZIKV$^{GFP}$ (MOI=2) and simultaneously treated with increasing concentrations of the compound **C1** or ternatin (ten-fold serial dilutions of considered compound). At 48 h post-infection, the percentage of GFP expressed by the cells was measured by FACS (Flow cytometric analysis of GFP fluorescence). Data represent the means $\pm$ SD of four independent experiments performed in triplicate and are expressed as relative values compared to mock-treated control.

Vero cells were infected with RRV$^{GFP}$ (MOI=0.1) and simultaneously treated with increasing concentrations of compound **C1** or ternatin (presence of ten-fold serial dilutions of considered compound). At 18 h post-infection, the percentage of GFP expressed by the cells is measured by FACS (Flow cytometric analysis of GFP fluorescence). Data represent the means $\pm$ SD of four independent experiments performed in triplicate and are expressed as relative values compared to mock-treated control.

Vero cells were infected with DENV (MOI = 0.1) and simultaneously treated with increasing concentrations of the compound **C1** or ternatin (presence of ten-fold serial dilutions of considered compound). At 72 h post-infection, the percentage of DENV-infected cells was measured by FACS assay. Data represent the means $\pm$ SD of three independent experiments performed in triplicate and are expressed as relative values compared to mock-treated control.

Results:

ZIKV antiviral activity for the compounds of formula (I) (**C1** and **C3** to **C7**) are gathered in **Table 5** herein after.

**Table 5.** ZIKV antiviral activity of compounds **C1** and **C3-C7** in comparison to ternatin.

| | **$IC_{50}$ (nM)** | | |
|---|---|---|---|
| | **Vero** | **A549** | **Huh7.5** |
| Compound **C1** | 9 | 4 | 2 |
| Compound **C3** | 9 | 0.50 | 0.003 |
| Compound **C4** | 163 | 26 | 12 |
| Compound **C5** | 244 | 40 | 11 |
| Compound **C6** | 115 | 13 | 9 |
| Compound **C7** | 176 | 46 | 16 |
| Ternatin | 584 | 92 | 23 |

Said results show that said compounds exert antiviral activity against ZIKV, at even better extend than the comparative ternatin concentrations.

Compound **C1** exerts potent antiviral activity against ZIKV (**Figure 9A**), RRV (**Figure 9B**) and DENV (**Figure 9C**) with $IC_{50}$ of 60, 57 and 42 nM, respectively, while ternatin showed a tenfold lower antiviral effect with $IC_{50}$ values

of 860, 977 and 493 nM against ZIKV, RRV and DENV respectively.

2. Antiviral activity on Coronaviruses

Protocols:

The effect of the compounds of formula (I) (**C1** and **C3** to **C7**) was evaluated on human alphacoronavirus HCoV-229-E. Huh7 cells were infected with HCoV-229E-Luc (MOI=0.01) and treated simultaneously with increasing concentrations of the compounds of formula (I) (ten-fold serial dilutions). At 24 h post-infection, luciferase activity was assessed following cell lysis. Data represent the means ± SD of three independent experiments performed in triplicate and are expressed as relative values compared to mock-treated control (**Figure 10A**).

The compound **C1** has been tested on the betacoronavirus SARS-CoV-2. Huh7 cells were infected with SARS-CoV-2 (MOI=0.01) and simultaneously treated with increasing concentrations of the compound of formula (I). At 24 h post-infection, cell supernatant was recovered for viral titration (**Figure 10B**). Viral progeny was measured by a PFU assay (plaque-forming assay) by quantifying infectious particles in the supernatant (PFU/mL).

Results:

HCoV-229E antiviral activity for the compounds of formula (I) (**C1** and **C3** to **C7**) are gathered in **Table 6** herein after.

**Table 6.** HCoV-229E-Luc antiviral activity of compounds **C1** and **C3** to **C7** in comparison to ternatin.

| | $IC_{50}$ (nM) Huh7 |
|---|---|
| Compound **C1** | 5.0 |
| Compound **C3** | 2.4 |
| Compound **C4** | 96.8 |
| Compound **C5** | 98.5 |
| Compound **C6** | 36.8 |
| Compound **C7** | 15.0 |
| Ternatin | 157.0 |

Said results show that said compounds exert potent antiviral activity on coronaviruses, at even better extend than the comparative ternatin does.

Compound **C1** exerts potent antiviral activity against HCoV-229E with $IC_{50}$ of 5 nM, while ternatin showed a much lower antiviral effect with $IC_{50}$ 157 nM (**Figure 10A**). Results presented in **Figure 10B** show that compound **C1** reduces SARS-CoV-2 viral progeny production in Huh7 cells by 3 logs at 0.6 nM.

3. Time of drug-addition assay

Time of drug-addition assay aims at characterizing the antiviral action of the compounds of the invention on various stages of the dissecting virus infectious cycle. These experiments were conducted with compound **C1**.

Compound **C1** (10 nM) or vehicle were used for treatment of A549 cells for viral inactivation assay, concurrently to virus input (Entry), after virus exposure (Replication) with specific washing steps and incubation periods (**Figure 11A**). The black or white arrows indicate respectively the presence or the absence of compound **C1** during the infection. GFP-expression in ZIKV-infected A549 cells under these conditions is presented in **Figure 11B.**

In an additional experiment, A549 cells were incubated 1 h with $ZIKV^{GFP}$ at 4 °C and temperature was shifted to 37 °C in absence (vehicle) or presence of 10 nM of compound **C1** which has been added at different time points post temperature shift. The data represent the means ± SD of four independent experiments performed in triplicate, and are expressed as relative values compared to vehicle-treated cells. Results shown in **Figure 11C** confirmed that compound **C1** targets late stages of viral infection.

4. Preclinical model

The compound **C1** was evaluated on a preclinical model in human primary bronchial epithelial cells (Mucilair) against SARS-CoV-2 (T. Meunier et al., Antimicrob Agents Chemother. 2022 Feb; 66(2): e01581-21). Mucilair cells were inoculated with SARS-CoV-2 in the presence of compound **C1** at 0.75, 7.5 or 75 pM for 1 h at the apical side. Inoculum was removed 1 h post-infection. At 72 h post-inoculation, cells were lysed to extract RNA. Viral RNA was quantified by qRTPCR. For RNA quantification, data are expressed relative to the Mock-treated control.

At a concentration of 75 pM, the compound **C1** shows a complete inhibition (>98%) of the infection by SARS-CoV-2 as shown in **Figure 12.**

5.1 Cellular cytotoxicity

Cytotoxicity of the compounds of formula (I) was evaluated on A549, Vero E6 and Huh7 cells by measuring the mitochondrial activity after 48 h of treatment by an MTT assay.

The results are gathered in **table 7** hereinafter.

**Table 7.** Cytotoxic activity on Vero E6, A549 and Huh7 cells of compounds **C1** and **C3-C7** in comparison to ternatin.

| | **$CC_{50}$ (nM)** | | |
|---|---|---|---|
| **Compounds** | **Vero** E6 | **A549** | **Huh7** |
| Compound **C1** | 200.0 | 11.2 | 4.9 |
| Compound **C3** | 411.6 | 0.0006 | 0.0001 |
| Compound **C4** | 3763.5 | 52.84 | 9.351 |
| Compound **C5** | 4814.5 | 99.8 | 31.88 |
| Compound **C6** | 3530.5 | 41 | 22.07 |
| Compound **C7** | 378.8 | 80.57 | 93.88 |
| Ternatin | 3217.5 | 191.9 | 857.4 |

*5.2. In vivo* studies in a zebrafish model

*In vivo* experiment in a zebrafish model (J. Haddad et al., Molecules 2020 May 15;25(10):2316. doi: 10.3390/molecules25102316) was performed with compound **C1**.

**Figure 13** retrieves the results obtained showing that compound **C1** did not impair fine-tune locomotor activity of zebrafish: (A) Inactivation time (<4 $mm.s^{-1}$). (B) Time of small activity (4-8 $mm.s^{-1}$). (C) Time of large activity (>8 $mm.s^{-1}$). (D) Total distance travelled at 1 day post injection. Control fish were injected with vehicle (PBS) and treated ones were injected with compound **C1** (10 ng/$\mu$L). After injection, fish were observed during 5 days. Statistical analyses were conducted using unpaired t-test comparisons, n = 5 (n: number of fishes per group).

Said experiment showed that compound **C1** has no toxicity nor impact on locomotion activity of adult zebrafish at the effective antiviral dose.

6. Conclusion on biological activity

The compounds of formula (I) are suitable for treating and/or preventing cancer and virus infections, in particular RNA virus infections caused by RNA viruses of group IV, more particularly, RRV, Dengue, Zika, HCoV-229E and SARS-CoV2 virus infections.

**Claims**

**1.** A compound of formula (I):

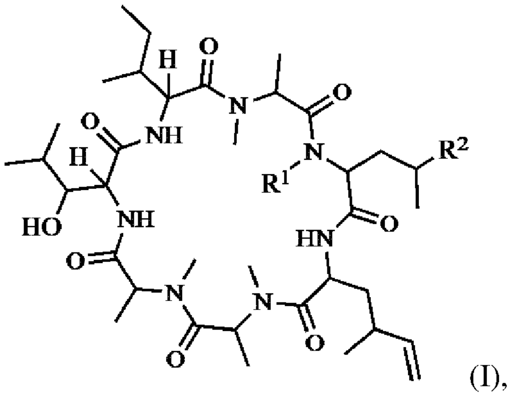

(I),

wherein it is selected from the following compounds:

- compound **C1** wherein $R^1$ is a methyl group and $R^2$ is a methyl group,
- compound **C2** wherein $R^1$ is hydrogen atom and $R^2$ is a methyl group,
- compound **C3** wherein $R^1$ is a methyl group and $R^2$ is an ethyl group,
- compound **C4** wherein $R^1$ is a methyl group and $R^2$ is a hydroxymethyl group,
- compound **C5** wherein $R^1$ is a methyl group and $R^2$ is a 1-hydroxyethyl group,
- compound **C6** wherein $R^1$ is a methyl group and $R^2$ is a hydroxyformyl methyl group, and
- compound **C7** wherein $R^1$ is a methyl group and $R^2$ is a 1-hydroxyformyl ethyl group.

**2.** Process for isolation of the compound of formula (I) as defined in claim 1, wherein they are extracted from species selected from the genera *Comptonella, Dutaillyea* and *Medicosma,* in particular from *Comptonella drupacea,* more particularly from their bark, leaves and/or fruits, for example bark.

**3.** Process according to claim 2, wherein the extraction is performed with a solvent such as ethyl acetate, dichloromethane, methanol, ethanol, acetone, diethyl ether or mixture thereof, in particular ethyl acetate, optionally followed by a concentration step.

**4.** Process according to claim 3, wherein the extract as obtained in claim 3 is submitted to an isolation step, in particular performed by using a silica gel column flash chromatography, in particular using a solvent selected from n-hexane, cyclohexane, *n*-heptane, dichloromethane, diethyl ether, methanol, ethanol and acetone for obtaining fractions or various polarities.

**5.** Process according to claim 4, wherein the most polar fractions as obtained in claim 4, in particular as obtained by using ethyl acetate and methanol, are purified by preparative liquid chromatography, such as High Performance Liquid Chromatography.

**6.** Process according to any of claims 2 to 5, wherein the extraction step is preceded by a drying step, in particular at a temperature equal or below 40 °C, in particular ranging from 30 to 40°C, followed by a grinding step.

**7.** A medicament, **characterized in that** it comprises the compound of formula (I) according to claim 1.

**8.** A pharmaceutical composition, **characterized in that** it comprises the compound of formula (I) according to claim 1 and at least one pharmaceutically acceptable excipient.

**9.** The compound of formula (I) according to claim 1, for use as a medicament.

**10.** The compound of formula (1) according to claim 1, for use in the prevention and/or treatment of cancer and of virus infections, in particular RNA virus infections, such as RNA virus infections from group IV or V, in particular infections caused by *picornaviruses* virus family encompassing the Hepatitis A virus, the enteroviruses, rhinoviruses, the poliovirus, and foot-and-mouth virus, by *flaviviruses* virus family encompassing the dengue virus, the yellow fever virus, the West Nile virus, the Japanese encephalitis virus, the Zika virus, the Usutu virus and the Tick-Borne encephalitis virus, *alphaviruses* virus family encompassing the Chikungunya virus, the Mayaro virus, the Ross River virus, and the Venezuelan Equine Encephalitis virus, by *hepaciviruses* virus family encompassing the Hepatitis C

virus, by *hepeviruses* virus family encompassing the Hepatitis E virus, by *coronaviruses* virus family encompassing HCoV, such as HCoV-229E, and SARS virus, such as the SARS-CoV and SARS-CoV-2, by measles virus, by *Filoviridae* virus family encompassing the Ebola virus, by *Paramyxoviridae* virus family encompassing the Respiratory Syncytial virus (RSV), by *Rhabdoviridae* virus family or y *Orthomyxoviridae* virus family encompassing the Influenzavirus A, Influenzavirus B and Influenzavirus C, and more particularly infections caused by Zika (ZIKV), Dengue (DENV), West Nile virus (WNV), Japanese encephalitis, Tick-borne encephalitis, Chikungunya (CHIKV), Ross River (RRV), Mayaro, measles virus or SARS-CoV-2.

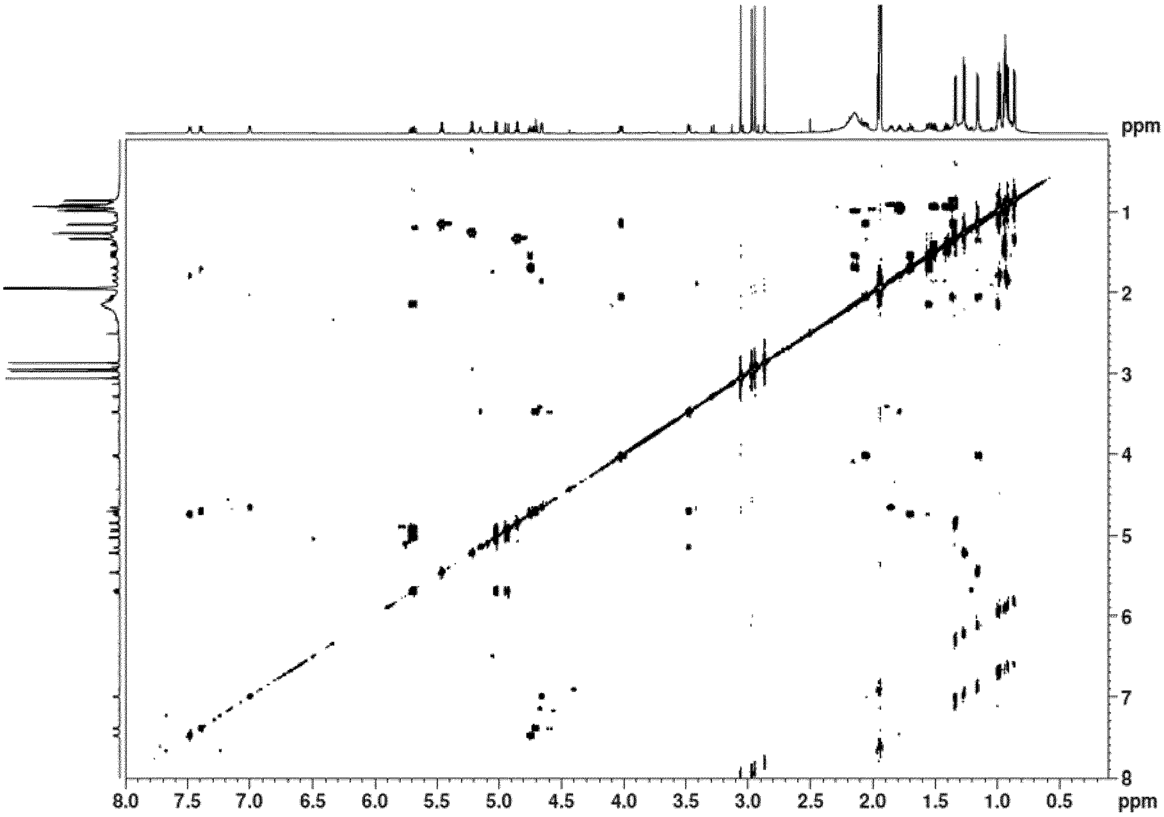
ppm
1
2
3
4
5
6
7
8
8.0
7.5
7.0
6.5
6.0
5.5
5.0
4.5
4.0
3.5
3.0
2.5
2.0
1.5
1.0
0.5
ppm

FIG 1

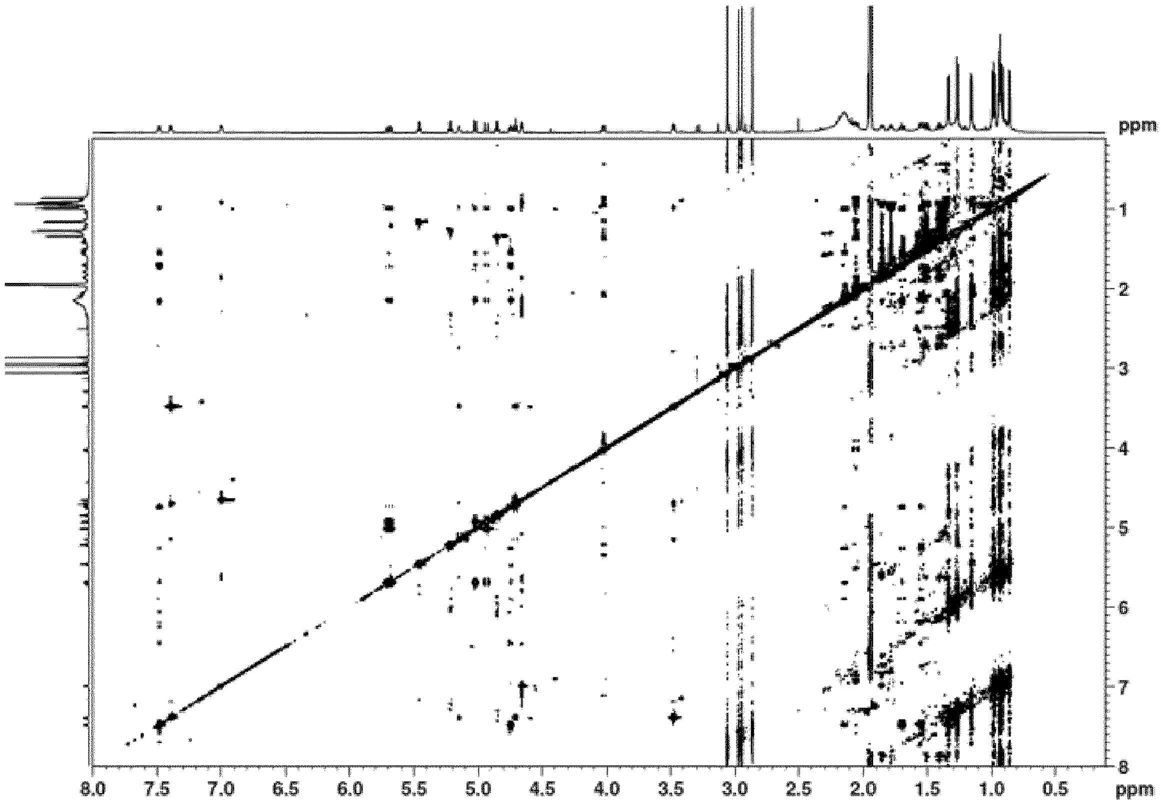
ppm
1
2
3
4
5
6
7
8
8.0
7.5
7.0
6.5
6.0
5.5
5.0
4.5
4.0
3.5
3.0
2.5
2.0
1.5
1.0
0.5
ppm

FIG 2

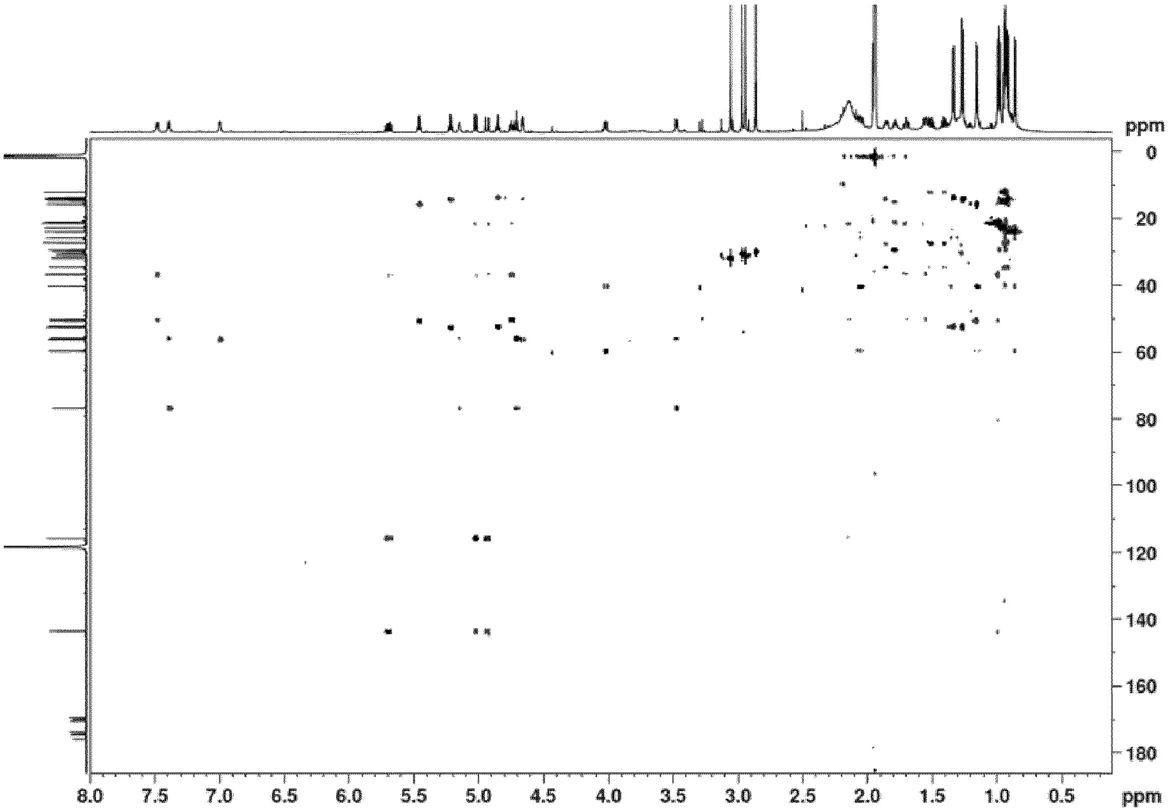
ppm
0
20
40
60
80
100
120
140
160
180
8.0
7.5
7.0
6.5
6.0
5.5
5.0
4.5
4.0
3.5
3.0
2.5
2.0
1.5
1.0
0.5
ppm

FIG 3

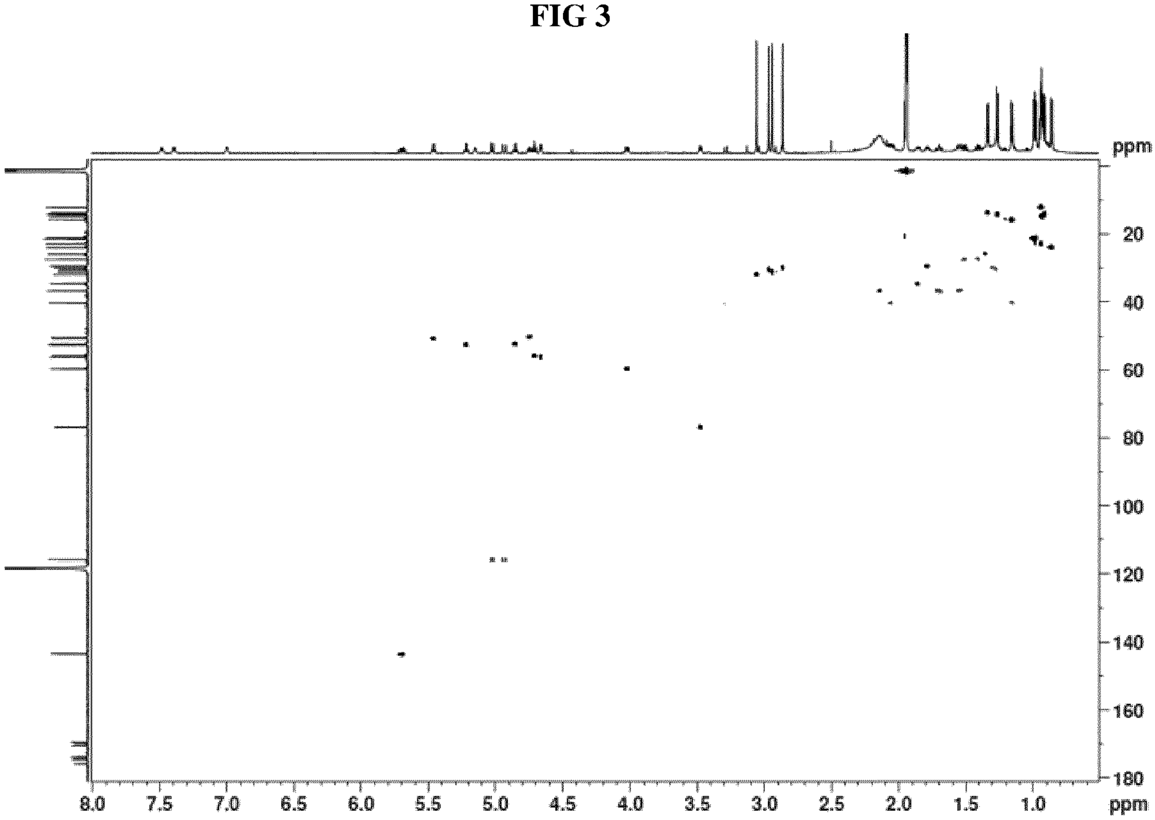
ppm
20
40
60
80
100
120
140
160
180
8.0
7.5
7.0
6.5
6.0
5.5
5.0
4.5
4.0
3.5
3.0
2.5
2.0
1.5
1.0
ppm

FIG 4

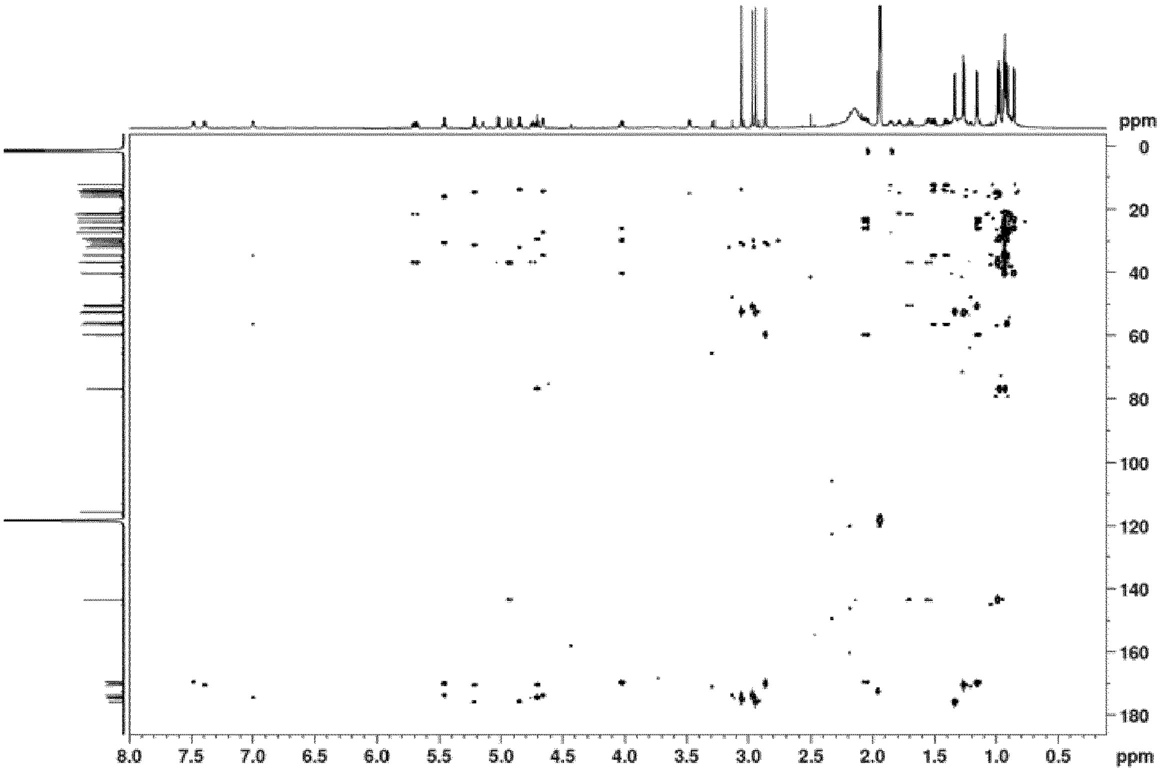
ppm
0
20
40
60
80
100
120
140
160
180
8.0
7.5
7.0
6.5
6.0
5.5
5.0
4.5
4.0
3.5
3.0
2.5
2.0
1.5
1.0
0.5
ppm

FIG 5

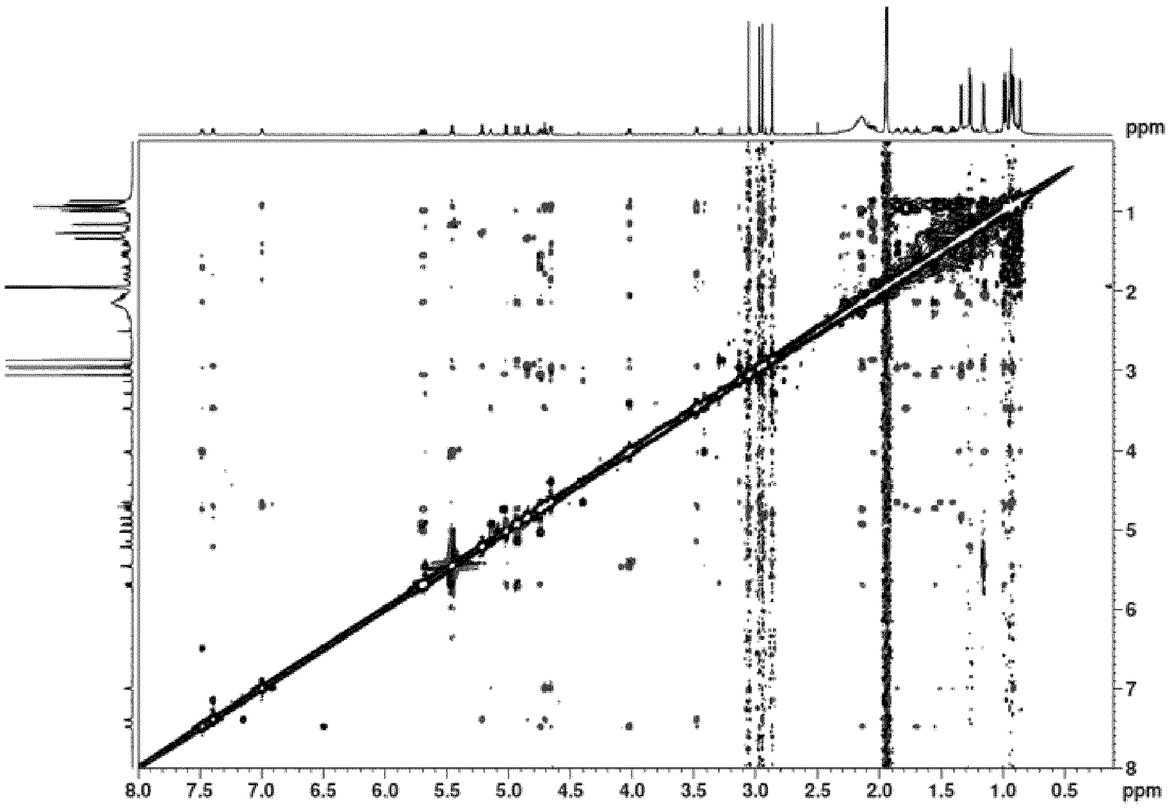
ppm
1
2
3
4
5
6
7
8
8.0
7.5
7.0
6.5
6.0
5.5
5.0
4.5
4.0
3.5
3.0
2.5
2.0
1.5
1.0
0.5
ppm

FIG 6

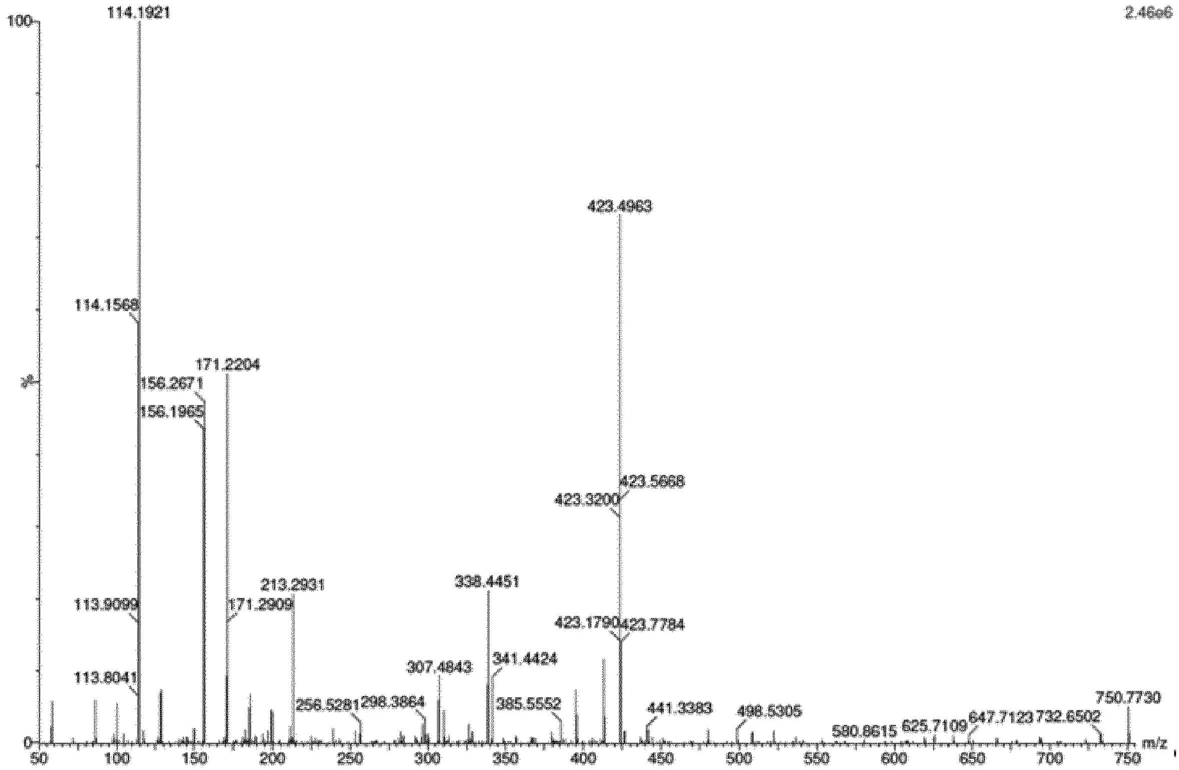
100
%
0
114.1921
2.46e6
423.4963
114.1568
171.2204
156.2671
156.1965
423.5668
423.3200
213.2931
338.4451
113.9099
171.2909
423.1790
423.7784
307.4843
341.4424
113.8041
256.5281
298.3864
385.5552
441.3383
498.5305
750.7730
580.8615
625.7109
647.7123
732.6502
m/z
50
100
150
200
250
300
350
400
450
500
550
600
650
700
750

FIG 7

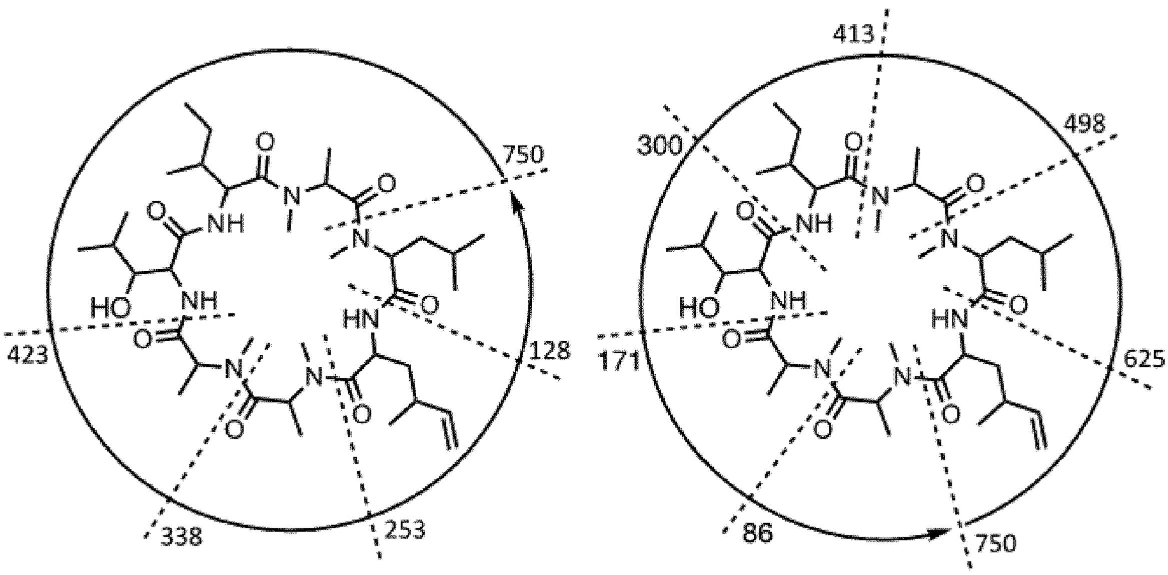
750
128
253
338
423
413
498
300
625
171
86
750

FIG 8

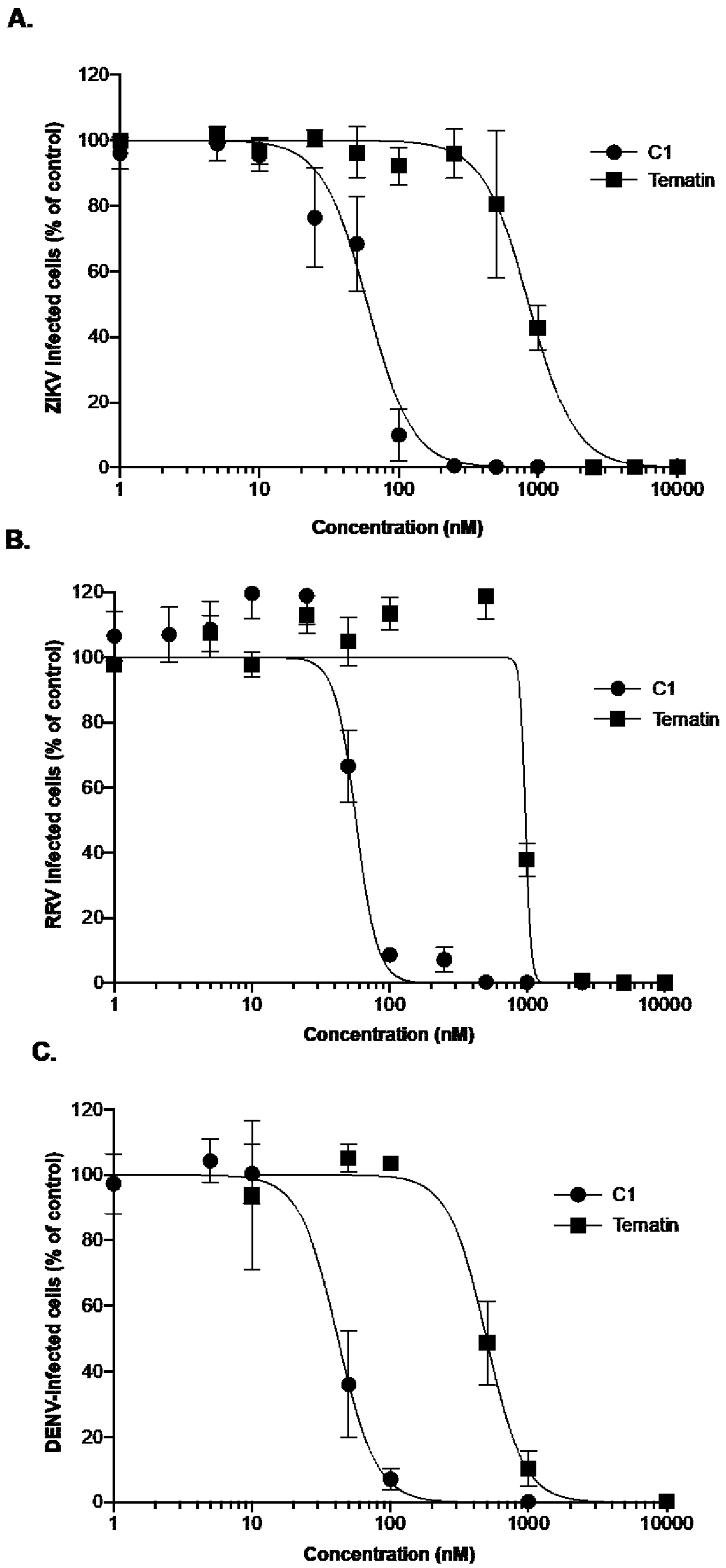
A.
ZIKV Infected cells (% of control)
120
100
80
60
40
20
0
1
10
100
1000
10000
Concentration (nM)
C1
Tematin
B.
RRV Infected cells (% of control)
120
100
80
60
40
20
0
1
10
100
1000
10000
Concentration (nM)
C1
Tematin
C.
DENV-Infected cells (% of control)
120
100
80
60
40
20
0
1
10
100
1000
10000
Concentration (nM)
C1
Tematin

FIG 9

A.

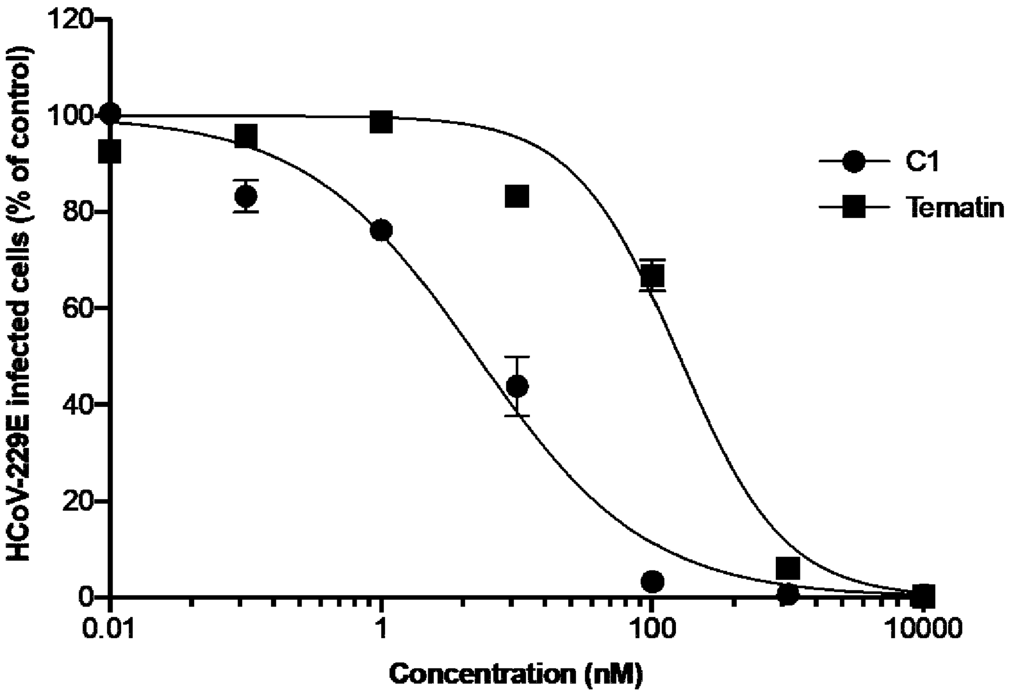


| C1 | Tematin |
|---|---|
| 5.076 | 157.1 |

B.

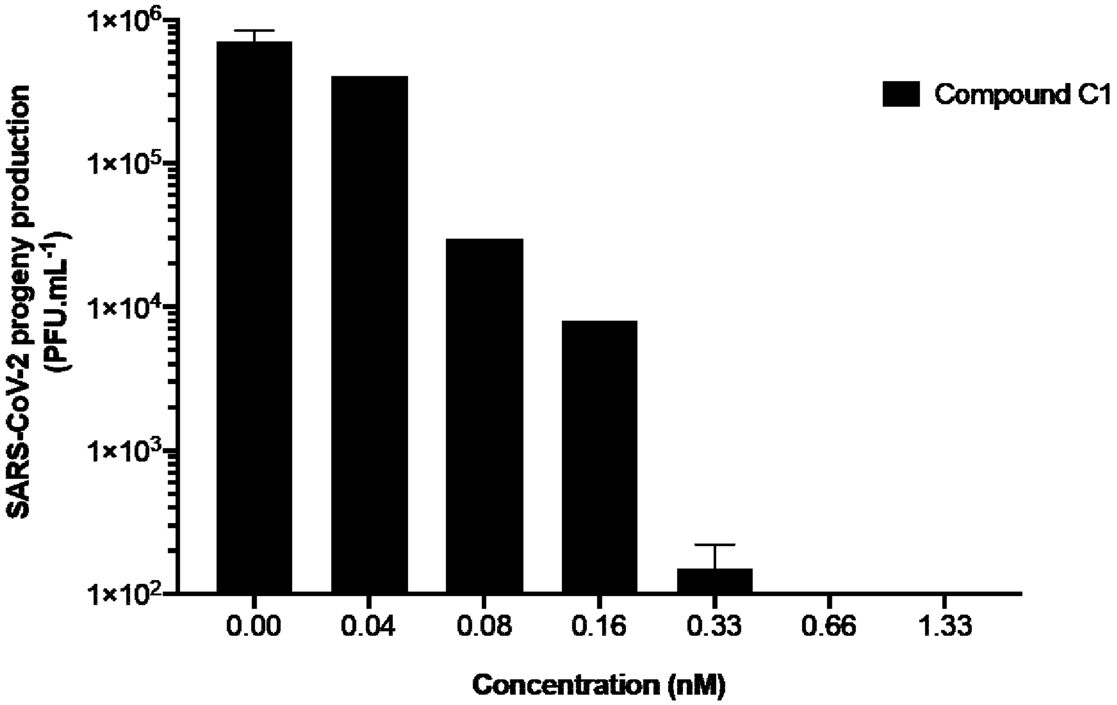


FIG 10

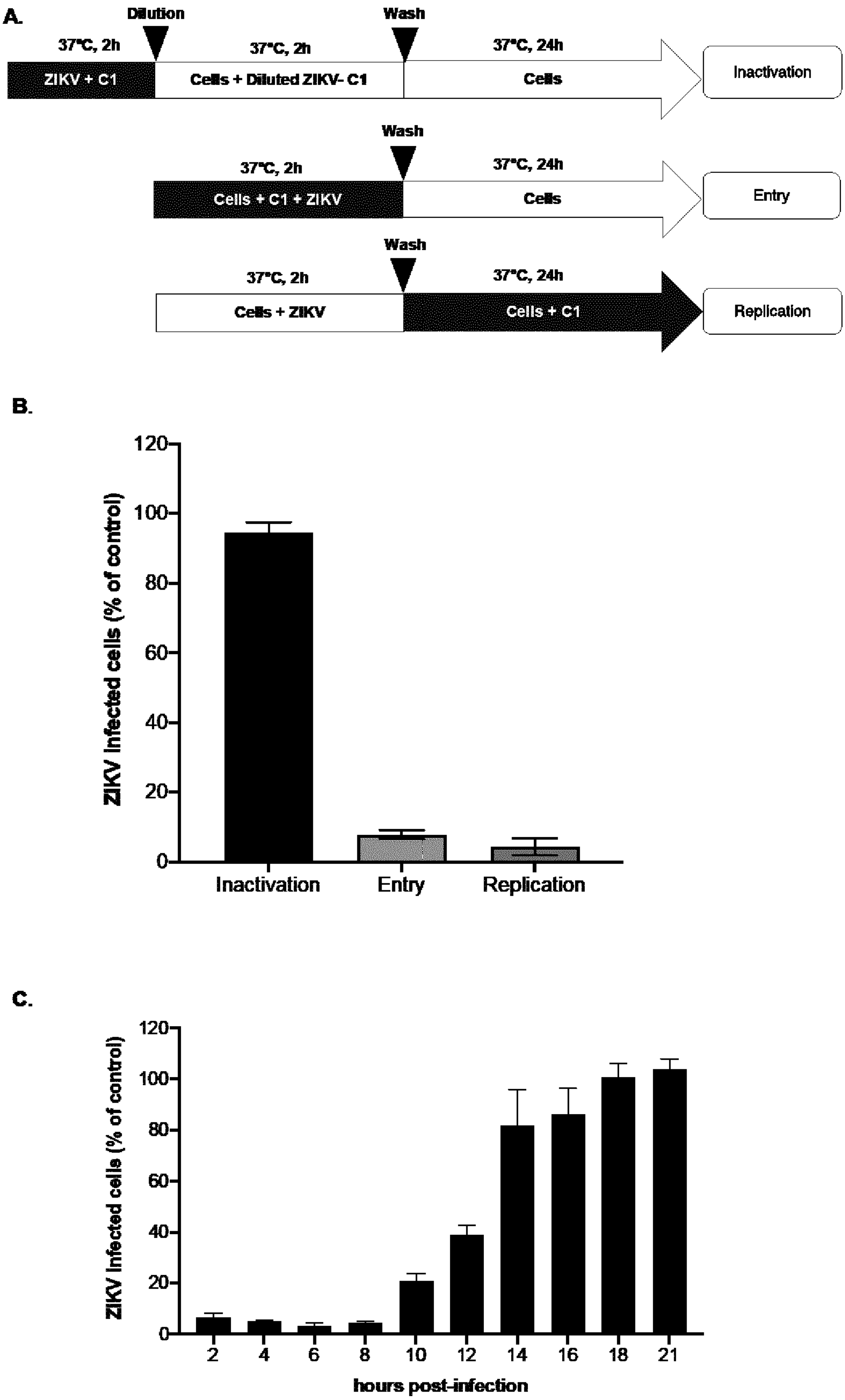
A.
Dilution
Wash
37°C, 2h
37°C, 2h
37°C, 24h
ZIKV + C1
Cells + Diluted ZIKV- C1
Cells
Inactivation
Wash
37°C, 2h
37°C, 24h
Cells + C1 + ZIKV
Cells
Entry
Wash
37°C, 2h
37°C, 24h
Cells + ZIKV
Cells + C1
Replication
B.
ZIKV Infected cells (% of control)
120
100
80
60
40
20
0
Inactivation
Entry
Replication
C.
ZIKV Infected cells (% of control)
120
100
80
60
40
20
0
2
4
6
8
10
12
14
16
18
21
hours post-infection

FIG 11

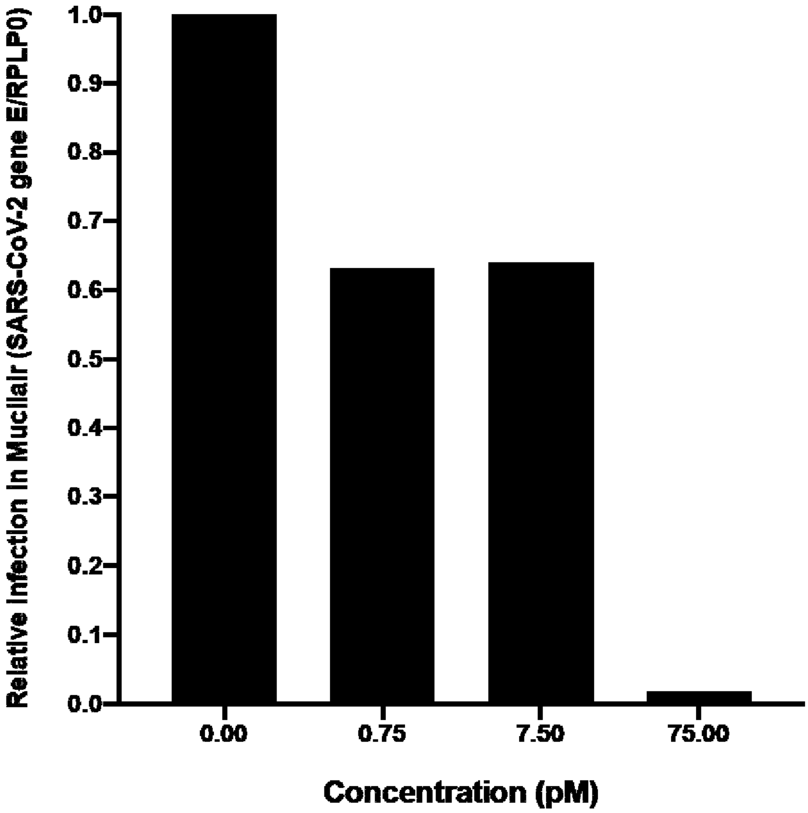
Relative Infection in Mucilair (SARS-CoV-2 gene E/RPLP0)
1.0
0.9
0.8
0.7
0.6
0.5
0.4
0.3
0.2
0.1
0.0
0.00
0.75
7.50
75.00
Concentration (pM)

FIG 12

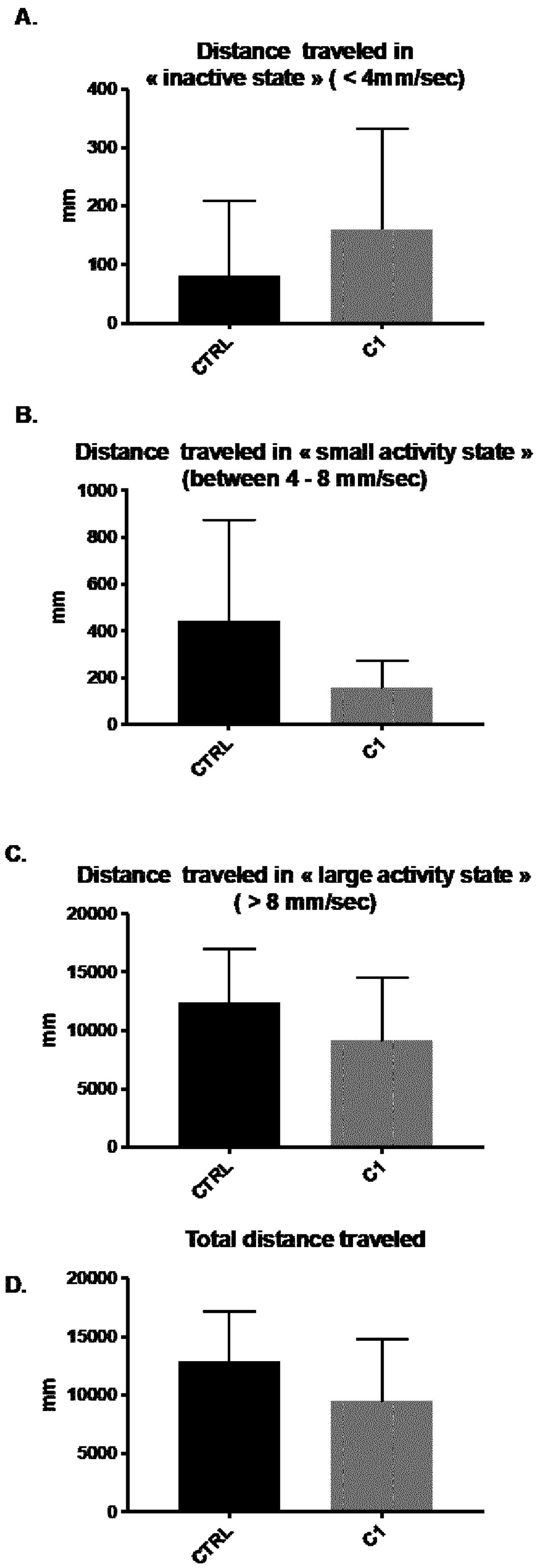
A.
Distance traveled in
« inactive state » ( < 4mm/sec)
mm
400
300
200
100
0
CTRL
C1
B.
Distance traveled in « small activity state »
(between 4 - 8 mm/sec)
mm
1000
800
600
400
200
0
CTRL
C1
C.
Distance traveled in « large activity state »
( > 8 mm/sec)
mm
20000
15000
10000
5000
0
CTRL
C1
Total distance traveled
D.
mm
20000
15000
10000
5000
0
CTRL
C1

FIG 13

Europäisches Patentamt
European Patent Office
Office européen des brevets

# EUROPEAN SEARCH REPORT

Application Number
EP 23 30 5746

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | WO 2010/062159 A1 (UNIV TEKNOLOGI MARA [MY]; CANTERPRISE LTD [NZ] ET AL.) 3 June 2010 (2010-06-03) * pages 31,52; table 11 * ----- | 1-10 | INV. A61P35/00 A61K38/00 C07K7/64 |
| Y | JP 2005 220074 A (FANCL CORP) 18 August 2005 (2005-08-18) * the whole document * ----- | 1-10 | |
| Y | SHIMOKAWA KENICHIRO ET AL: "Whole structure-activity relationships of the fat-accumulation inhibitor (-)-ternatin: recognition of the importance of each amino acid residue", JOURNAL OF MEDICINAL CHEMISTRY, AMERICAN CHEMICAL SOCIETY, US, vol. 51, no. 19, 9 October 2008 (2008-10-09), pages 5912-5914, XP002598747, ISSN: 0022-2623, DOI: 10.1021/JM800741N [retrieved on 2008-09-18] * page 5913; figure 2 * ----- | 1-10 | TECHNICAL FIELDS SEARCHED (IPC) |
| Y | US 2014/107018 A1 (ZHANG HONGJIE [HK]) 17 April 2014 (2014-04-17) * paragraphs [0136] - [0142]; tables 2,3 * ----- | 1-10 | A61P C07K A61K |

-/--

The present search report has been drawn up for all claims

| Place of search | Date of completion of the search | Examiner |
|---|---|---|
| Munich | 23 October 2023 | Pinheiro Vieira, E |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 23 30 5746

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CARELLI JORDAN D ET AL: "Ternatin and improved synthetic variants kill cancer cells by targeting the elongation factor-1A ternary complex", AUTHOR RESPONSE: TERNATIN AND IMPROVED SYNTHETIC VARIANTS KILL CANCER CELLS BY TARGETING THE ELONGATION FACTOR-1A TERNARY COMPLEX, vol. 4, 10 December 2015 (2015-12-10), page e10222, XP055848124, Retrieved from the Internet: URL:https://www.ncbi.nlm.nih.gov/pmc/ivip/2050-084X> | 1,7-10 | |
| Y | * figures 1,3 * | 2-6 | |
| | ----- | | |
| | | | TECHNICAL FIELDS SEARCHED (IPC) |

The present search report has been drawn up for all claims

| Place of search | Date of completion of the search | Examiner |
|---|---|---|
| Munich | 23 October 2023 | Pinheiro Vieira, E |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO.

EP 23 30 5746

This annex lists the patent family members relating to the patent documents cited in the above-mentioned European search report. The members are as contained in the European Patent Office EDP file on
The European Patent Office is in no way liable for these particulars which are merely given for the purpose of information.

23-10-2023

| Patent document cited in search report | Publication date | Patent family member(s) | Publication date |
|---|---|---|---|
| WO 2010062159 A1 | 03-06-2010 | EP 2352746 A1 | 10-08-2011 |
| | | US 2011201642 A1 | 18-08-2011 |
| | | WO 2010062159 A1 | 03-06-2010 |
| JP 2005220074 A | 18-08-2005 | NONE | |
| US 2014107018 A1 | 17-04-2014 | CN 104640873 A | 20-05-2015 |
| | | EP 2909227 A1 | 26-08-2015 |
| | | TW 201420608 A | 01-06-2014 |
| | | US 2014107018 A1 | 17-04-2014 |
| | | WO 2014059928 A1 | 24-04-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010062159 A


### Non-patent literature cited in the description


- *CHEMICAL ABSTRACTS,* 148619-41-4
- **J. D. CARELLI et al.** *eLife,* 2015, vol. 4, e10222
- Ullmann's Encyclopedia of Industrial Chemistry. Marcel Dekker, 1989
- **ANSEL et al.** Pharmaceutical Dosage Forms and Drug Delivery Systems. WILLIAMS & WILKINS, 1994
- **SHIMOKAWA et al.** Ternatin, a highly N-methylated cyclic heptapeptide that inhibits fat accumulation: structure and synthesis. *Tetrahedron Letters,* 2006, vol. 47, 4445-4448
- **T. MEUNIER et al.** *Antimicrob Agents Chemother.,* February 2022, vol. 66 (2), e01581-21
- **J. HADDAD et al.** *Molecules,* 15 May 2020, vol. 25 (10), 2316